# EUROPEAN PATENT SPECIFICATION

(11) **EP 0 692 011 B1**
(45) Date of publication and mention of the grant of the patent: **06.11.1996**
(21) Application number: 94913104.9
(22) Date of filing: 29.03.1994
(51) Int. Cl.: C10L 1/22, C07D 207/26, C08G 65/28, C10L 10/00

(54) **FUEL COMPOSITIONS**
KRAFTSTOFFZUSAMMENSETZUNGEN
COMPOSITIONS COMBUSTIBLES

(30) Priority: 30.03.1993 US 40245
(43) Date of publication of application: 17.01.1996
(73) Proprietor: SHELL INTERNATIONALE RESEARCH MAATSCHAPPIJ B.V., 2596 HR Den Haag (NL); SHELL CANADA LIMITED, Calgary, Alberta T2P 2H5 (CA)
(72) Inventor: LIN, Jiang-Jen, Houston, TX 77083 (US); MACIAS, James, Robert, Houston, TX 77449 (US); HAURY, Earl, Jon, Houston, TX 77099 (US); WEAVER, Sarah, Louise c/o Shell Oil Company, 900 Louisiana Houston, Texas 77002 (US); EDWARDS, Charles, Lee, Houston, TX 77077 (US); WANG, Pen-Chung, Houston, TX 77079 (US); VAPORCIYAN, Garo, Garbis, Houston, TX 77077 (US)
(86) International application number: PCT/EP94/01020
(87) International publication number: WO 94/22984

(56) References cited:
- EP-A- 0 582 209
- WO-A-88/06586
- FR-A- 1 500 644
- US-A- 3 337 585
- US-A- 4 247 301
- US-A- 4 298 708
- US-A- 4 397 750
- US-A- 4 760 152
- US-A- 4 958 032

## Description

This invention relates to fuel compositions containing amide derivatives, a method of operating an internal combustion engine using such fuel compositions, fuel additive concentrates, certain amide derivatives per se and their preparation.

It is well known in the art that internal combustion engines, more particularly spark ignition engines, tend to exhibit what is termed the octane requirement increase effect. This effect may be described as the tendency for an initially new or relatively clean engine to require higher octane quality fuel as operating time accumulates, and is coincidental with the formation of deposits in the region of the combustion chamber of the engine.

During initial operation of a new or clean engine, a gradual increase in octane requirement, i.e., fuel octane number required for knock-free operation, is observed with an increasing build up of combustion chamber deposits until a stable or equilibrium octane requirement level is reached. This level appears to correspond to a point in time when the quantity of deposit accumulation on the combustion chamber and valve surfaces no longer increases but remains relatively constant. This so-called "equilibrium value" is normally reached between 3,000 and 20,000 miles or corresponding hours of operation. The actual equilibrium value of this increase can vary with engine design and even with individual engines of the same design; however, in almost all cases, the increase appears to be significant, with octane requirement increase values ranging from about 2 to about 10 research octane numbers being commonly observed in modern engines.

Accumulation of deposits on the intake valves of internal combustion engines also presents problems. The accumulation of such deposits is characterized by overall poor driveability including difficulty in starting, tendency to stall, inclination to stumble during acceleration and rough engine idle.

Many additives are known which can be added to hydrocarbon fuels to prevent or reduce deposit formation, or remove or modify formed deposits, in the combustion chamber and on adjacent surfaces such as intake valves, ports, and spark plugs, which in turn causes a decrease in octane requirement.

Continued improvements in the design of internal combustion engines, e.g., fuel injection and carburetor engines, bring changes to the environment of such engines thereby creating a continuing need for new additives to control the problem of inlet system deposits and to improve driveability which is usually related to deposits.

US Patent 4,958,032 (o'Lenick) describes a class of lactam compounds of general formula 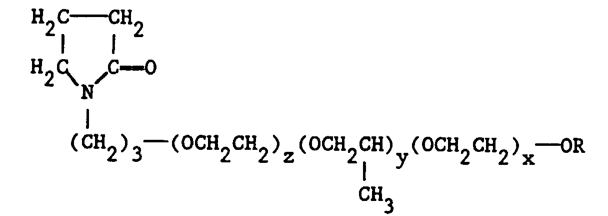 wherein;
R is an alkyl having from 10 to 36 carbon atoms;
x, y and z are independently integers from 0 to 50, with the proviso that the sum of x+y+z be greater than zero. These compounds are described as prime candidates for cosmetic applications, and particularly as hair conditioning agents.
US Patent 4,975,159 (Dahms, assigned to Schering A.G.) relates to aqueous acidic baths for electrodeposition of copper coatings wherein shininess and mechanical properties of the copper coatings are enhanced by inclusion in the bath of a substituted alkoxylated lactam which is advantageously of the general formula 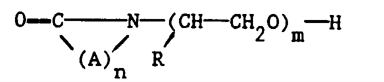 wherein R is methyl or hydrogen,

- A: is a hydrocarbon residue, preferably a -CH₂- group,
- n: is a whole number from 2 to 10, advantageously from 2 to 5, and
- m: is is a whole number from 1 to 50.

It has now been found that use in an internal combustion engine of fuel compositions containing certain cyclic amide alkoxylates can lead to control of octane requirement increase, and/or reduction of octane requirement, and/or reduction of intake valve deposits.

According to the present invention there is provided a fuel composition comprising a mixture of a major amount of hydrocarbons in the gasoline boiling range and a minor amount of an additive compound, more particularly a cyclic amide alkoxylate, having the general formula I: 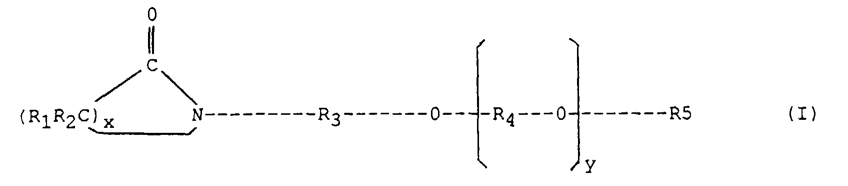 wherein x is from 2 to 20; y is from 1 to 50; R₁ and R₂ are independently hydrogen or optionally substituted hydrocarbyl of 1 to 100 carbon atoms; R₃ is optionally substituted hydrocarbyl of 1 to 100 carbon atoms; each R₄ is independently optionally substituted hydrcarbyl of 2 to 100 carbon atoms of the formula: 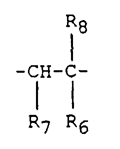 wherein R₆, R₇ and R₈ are each independently hydrogen or optionally substituted hydrocarbyl, provided that at least one of R7 and R8 is hydrogen, such that R₆ and R₈ together or R₆ and R₇ together contain 0 to 98 carbon atoms, or R₆ and R₈ or R₆ and R₇ together represent a hydrocarbyl bridge containing 3 to 98 carbon atoms; R₅ is hydrogen, or optionally substituted hydrocarbyl of 1 to 100 carbon atoms or acyl of 1 to 20 carbon atoms and the average formula weight of the additive compound is at least 600.

The term "average formula weight" corresponds to average relative molecular mass, i.e. the sum of the relative atomic masses of the constituent atoms of an average molecule. This value may be estimated from the relative quantities of reagents used in the preparation of the compound of formula I, or it may be assessed by an appropriate method of determination of number average molecular weight, to which it closely corresponds.

The average formula weight of the additive compound of formula I is preferably from 800 to 4000, more preferably from 800 to 2000, and advantageously from 1000 to 2000.

As used herein, the term "hydrocarbyl" represents a radical formed by the removal of one or more hydrogen atoms from a carbon atom of a hydrocarbon (not necessarily the same carbon atom). Useful hydrocarbyls are aliphatic, aromatic, substituted, unsubstituted, acyclic or cyclic. Preferably, the hydrocarbyls are aryl, alkyl, alkenyl or cycloalkyl and are straight-chain or branched chain. Representative hydrocarbyls include methyl, ethyl, butyl, pentyl, methylpentyl, hexenyl, ethylhexyl, dimethylhexyl, octamethylene, octenylene, cyclooctylene, methylcyclooctylene, dimethylcyclooctyl, isooctyl, dodecyl, hexadecenyl, octyl, eicosyl, hexacosyl, triacontyl and phenylethyl. When the hydrocarbyl is substituted, it contains a functional group such as carbonyl, carboxyl, nitro, amino, hydroxy (e.g. hydroxyethyl), oxy, cyano, sulfonyl, and sulfoxyl. The majority of the atoms, other than hydrogen, in substituted hydrocarbyls are carbon, with the heteroatoms (e.g., oxygen, nitrogen, sulfur) representing only a minority, 33% or less, of the total non-hydrogen atoms present.

Those skilled in the art will appreciate that functional groups such as nitro, amino (-NH₂), hydroxy and cyano in a substituted hydrocarbyl group will displace one of the hydrogen atoms of the hydrocarbyl, whilst functional groups such as carbonyl, carboxyl, amino 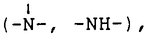 oxy, sulfonyl and sulfoxyl in a substituted hydrocarbyl group will displace a 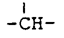 or -CH₂-moiety of the hydrocarbyl. In "optionally substituted hydrocarbyl of 1 to 100 carbon atoms", "1 to 100 carbon atoms" represents the total number of carbon atoms in the optionally substituted hydrocarbyl group.

When R₁ and/or R₂ are optionally substituted hydrocarbyl, they are each preferably optionally substituted hydrocarbyl of 1 to 20 carbon atoms, even more preferably, alkyl of 1 to 20 carbon atoms and most preferably alkyl of 1 to 8 carbon atoms. When R1 and/or R2 are hydrocarbyl of a relatively high number of carbon atoms, i.e., greater than about 50 carbon atoms, R₁ and/or R₂ may conveniently be represented by polymeric hydrocarbyls such as polyisobutylene, polybutene, polypropylene or polyalpha olefin.

Preferably, at least one of R₁ and R₂ is hydrogen. It is even more preferred for both of R₁ and R₂ to be hydrogen.

In formula I, x is from 2 to 20, preferably from 3 to 11. Examples of particularly preferred compounds of formula I are those in which x is 3, 5 or 11. X is preferably from 3 to 5, conveniently 3 or 5, and advantageously 3.

R3 is optionally substituted hydrocarbyl of 1 to 100 carbon atoms, preferably of 1 to 20 carbon atoms. When R₃ is hydrocarbyl of a relatively high number of carbon atoms, i.e., greater than about 50 carbon atoms, R₃ may conveniently be represented by polymeric hydrocarbyls such as polyisobutylene, polybutene, polypropylene or polyalphaolefin. Particularly preferred compounds are those in which R₃ is alkylene of 1 to 20 carbon atoms, more preferably alkylene of 2 to 10 carbon atoms, and most preferably alkylene of 2 to 4 carbon atoms, e.g. ethylene optionally substituted by a methyl or ethyl group.

In formula I, y is from 1 to 50, preferably from 8 to 40, more preferably from 8 to 30, e.g. from 9 to 29, and even more preferably from 18 to 24. Those of ordinary skill in the art will recognise that when the compounds of formula I are utilised in a composition, y will not have a single value applicable for each molecule but will instead be represented by a range of different values. As used in this specification, y is considered to be a (number) average of the various values of y that are found in a given composition, which number has been rounded to the nearest integer. The breadth of the range of individual values of present in a given composition is indicated by the polydispersity (polydispersity = weight average molecular weight divided by the number average molecular weight).

Each R₄ as defined above is independently optionally substituted hydrocarbyl of 2 to 100 carbon atoms, preferably of 2 to 20 carbon atoms, more preferably of 2 to 14 carbon atoms and may advantageously be four carbon atoms. When R₄ is hydrocarbyl of a relatively high number of carbon atoms, i.e., greater than about 50 carbon atoms, R₄ may conveniently be represented by polymeric hydrocarbyls such as polyisobutylene, polybutene, polypropylene or polyalpha olefin.

R4 in the compounds of formula I is hydrocarbyl (geminal or vicinal) of the formula: 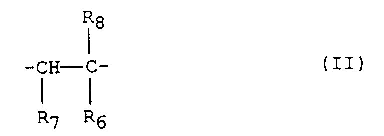 wherein at least one of R₇ and R₈ is hydrogen, i.e. R₄ is of formula: 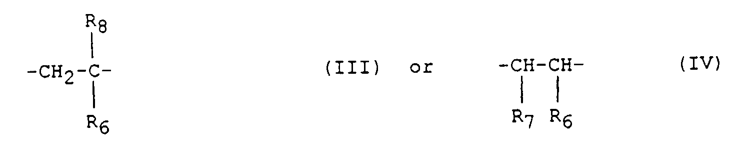 wherein R₆, R₇ and R₈ are each independently hydrogen or optionally substituted hydrocarbyl, as defined hereinbefore, such that R₆ and R₈ together or R₆ and R₇ together contain 0 to 98 carbon atoms, or R₆ and R₈ or R₆ and R₇ together represent a hydrocarbyl bridge containing 3 to 98 carbon atoms. Preferably, each of R₆, R₇ and R₈ is independently hydrogen or optionally substituted hydrocarbyl of 1 to 18 carbon atoms. R₇ and R₆, or alternatively R₆ and R₈, may be taken together to form a divalent linking hydrocarbyl group of 3 to 12 carbon atoms.

The most preferred compounds of formula I are those in which R₄ is hydrocarbyl as represented by formula III above wherein R₈ is hydrogen and R₆ is independently hydrogen, alkyl of 1 to 18 carbon atoms or oxy-substituted hydrocarbyl of 1 to 18 carbon atoms, particularly those compounds where R₈ is hydrogen and each R₆ is independently alkyl of 1 to 2 carbon atoms, more especially those wherein x is from 3 to 5, R₁ and R₂ are independently hydrogen or hydrocarbyl of 1 to 8 carbon atoms, R₃ is hydrocarbyl of 2 to 4 carbon atoms and R₅ is hydrogen.

When one or more moieties R₆ are oxy-substituted hydrocarbyl of 1 to 18 carbon atoms, each R₆ is preferably independently selected from alkoxy-substituted alkylene of 1 to 18 carbon atoms and aryloxy-substituted alkylene of 1 to 18 carbon atoms. Particularly preferred alkoxy-substituted alkylene groups include ethylhexyleneoxymethylene, isopropoxymethylene, and butoxymethylene. Particularly preferred aryl-substituted alkylene groups include nonylphenoxymethylene and phenoxymethylene.

When y is greater than 1, the individual R₄'s are the same or different. For example, if y is 20, each R₄ can be alkylene of four carbon atoms. Alternatively, the R₄'s can differ and for instance, independently be alkylene from two to four carbon atoms. When the R₄'s differ, they may be present in blocks, i.e., all groups in which R₄ is alkyl of three carbon atoms will be adjacent, followed by all groups in which R₄ is alkyl of two carbon atoms, followed by all groups in which R₄ is alkyl of four carbon atoms. When the R₄'s differ, they may also be present in any random distribution.

R₅ is hydrogen, hydrocarbyl, as defined hereinbefore, of 1 to 100 carbon atoms or acyl of 1 to 20 carbon atoms. Preferably, R₅ is hydrogen. When R₅ is hydrocarbyl, it is preferably of 1 to 70 carbon atoms, more preferably of 1 to 20 carbon atoms. The term "acyl", as used herein, represents the residue of an organic acid in which the -OH of a carboxyl group is removed to form R₉CO-. R₉ is monovalent hydrocarbyl, as defined hereinbefore, of 2 to 19 carbon atoms, preferably alkyl or aryl of 2 to 19 carbon atoms. Representative acyl groups include acetyl-, butyryl-, caproyl-, acrylyl-, benzoyl- and methacrylyl-. Substituted acyl groups are also contemplated. Substituted acyl groups contain a functional group such as those described in relation to substituted hydrocarbyl groups.

The invention further provides novel compounds of formula I, wherein x, y, R₁, R₂, R₃, R₄ and R₅ are as defined above, and R₃ or at least one R₄ moiety is optionally substituted hydrocarbyl of at least four carbon atoms. Preferably x is from 2 to 4, most preferably 3. y is greater than 1, at least R₃ or one R₄ must have at least four carbon atoms. R₅ is as defined hereinbefore.

It is preferred that the compounds of formula I also have a weight average molecular weight (as determined by gel permeation chromatography (GPC)) of at least 600. Preferably, the weight average molecular weight is from about 800 to about 4000, even more preferably from about 1000 to about 2000.

Typical compounds represented by formula I include those listed by structure in Table 1:

### TABLE 1

For purposes of clarity hydrogen atoms have been omitted from the ring structures of Table 1 and a standard schematic structure for a benzene ring (without hydrogen and carbon atoms) has been used. 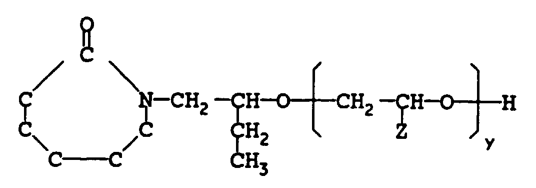 wherein y is from 18 to 24 and Z is independently H, -CH₃ or -CH₂CH₃ including mixtures thereof. 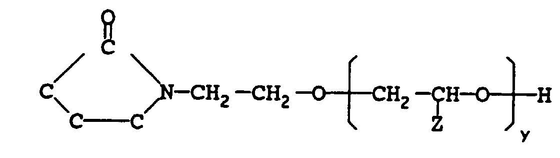 wherein y is from 18 to 24 and Z is independently H, -CH₃ or -CH₂CH₃ including mixtures thereof. 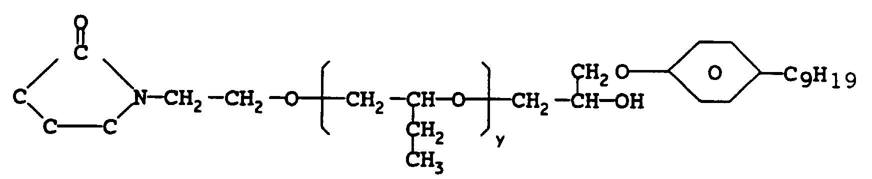 wherein y is from 8 to 40. 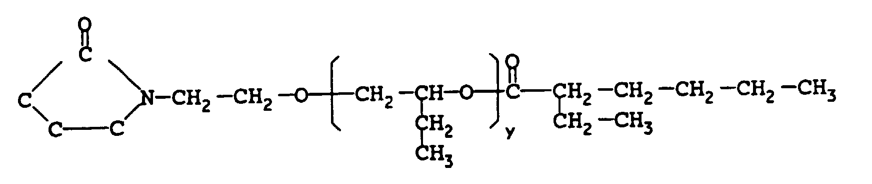 wherein y is from 8 to 40. 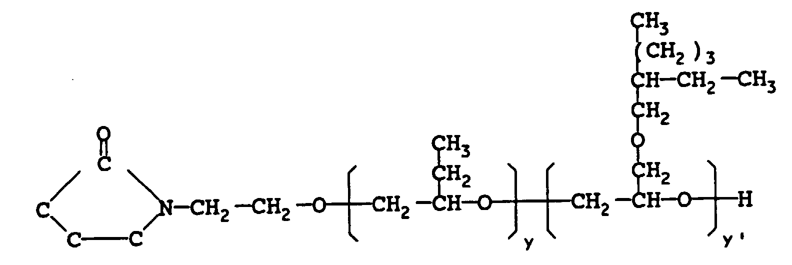 wherein the sum of y and y' is from 1 to 50. 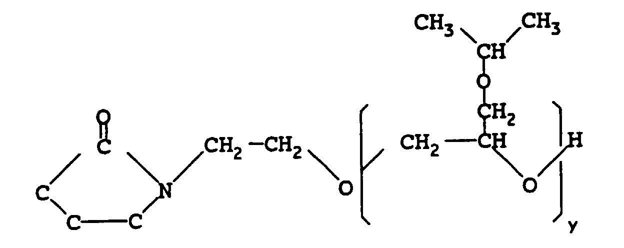 wherein y is from 8 to 40.

Compounds of formula I as defined above may be prepared in accordance with the present invention by a process which comprises reacting a compound of general formula XI 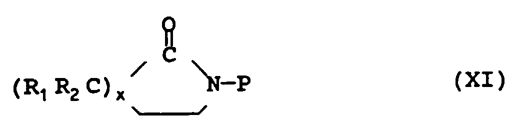 wherein x, R₁ and R₂ are as defined above and P is hydrogen or a group of formula -R₃-OH, wherein R₃ is as defined above with at least one cyclic ether, preferably epoxy, compound which upon ring opening leaves a moiety -R₄-O-, provided that when P is hydrogen, the compound of formula XI is reacted with at least one mol of cyclic ether, preferably epoxy, compound which leaves a moiety -R₃-O- upon ring opening per mol of compound of formula XI, to form a compound of formula I wherein R₅ is hydrogen, optionally followed by etherification or esterification to form a compound of formula I wherein R₅ is optionally substituted hydrocarbyl of 1 to 100 carbon atoms or acyl of 1 to 20 carbon atoms.

Reaction of the compound of formula XI with the cyclic ether, preferably epoxy, compound may conveniently be effected in the presence of a basic alkali metal compound, for example a potassium compound such as potassium hydroxide, potassium hydride or a potassium alkoxide, such as potassium t-butoxide.

In one embodiment, compounds of formula I may be prepared utilizing cyclic amidoalcohol compounds of formula XI wherein P is a group of formula -R₃-OH, i.e. cyclic amidoalcohol compounds of general formula:

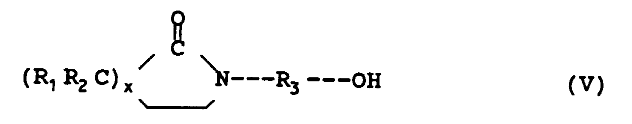

Illustrative examples of such cyclic amidoalcohol compounds initiators include N-(2-hydroxyethyl)-pyrrolidinone, N-[2-(2-hydroxyethoxy)ethyl]-pyrrolidinone, N-(2-hydroxypropyl)-pyrrolidinone and N-(2-hydroxy-2-methyl-ethyl)-pyrrolidinone, with N-(2-hydroxyethyl)-pyrrolidinone being the most preferred.

Certain of these cyclic amidoalcohol compounds are available commercially, such as, N-(2-hydroxyethyl)-pyrrolidinone (obtained from International Specialty Products or Aldrich Chemical Company). Cyclic amidoalcohol compounds may be prepared by any of the methods known and described in the art, for example by the method of Puetzer et al in J. Am. Chem. Soc. 74 4959 (1952), by reacting a lactone of the formula: 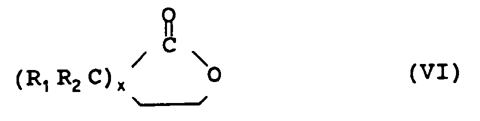 with an aminoalcohol of the formula:

NH₂-R₃-OH (VII)

wherein R₁, R₂, R₃ and x are as defined above. Illustrative lactones for use in making the cyclic amidoalcohol compounds include: α-methyl-γ-butyrolactone, β-methyl-γ-butyrolactone, ε-caprolactone, γ-caprolactone, γ-phenyl-γ-butyrolactone, phthalide, 3,3a,6,6a-tetrahydro-2H-cyclopenta[b]furan-2-one, tetronic acid (tetrahydrofuran-2,6-dione), β-propiolactone and isochromanone. Illustrative aminoalcohols for reacting with lactones to make the cyclic amidoalcohol compounds include: 2-amino-1-butanol, 2-(2-aminoethoxy) ethanol, 2-amino-2-ethyl-1,3-propanediol, 2-amino-2-methyl-1-propanol, 3-amino-1-propanol and 1-amino-2-propanol.

In an alternative embodiment, compounds of formula I may be prepared by reacting with the cyclic ether, preferably epoxy compound a cyclic amide of the general formula: 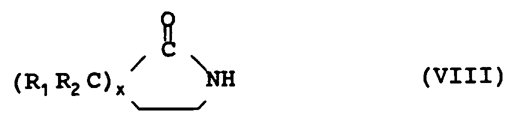 wherein R₁, R₂ and x are as defined above. Illustrative examples of cyclic amides which can be employed include cycloalkyl lactams wherein R₁ and R₂ are both hydrogen and x is 2, 3 (butyrolactam), 4, 5 (caprolactam), 6 and 7, with the compounds wherein x is 2, 3 and 5.

Certain cyclic amides are also available commercially, such as, ε-caprolactam (ε-hexanolactam available commercially from Aldrich Chemical Company), 2-pyrrolidinone (pyrrolidone or γ-butyrolactam available commercially from Aldrich Chemical Company) and laurolactam (2-azacyclotridecanone available commercially from Aldrich Chemical Company) with ε-caprolactam being the most preferred.

The cyclic ether, preferably epoxy, compounds which upon ring opening leave a moiety -R₄-O- contain from 2 to 100 carbon atoms, preferably from 2 to 20 carbon atoms, more preferably from 2 to 14 carbon atoms, and most preferably four carbon atoms. The cyclic ether compounds may be internal epoxides such as 2,3 epoxides of the formula: 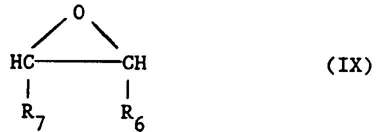 wherein R₆ and R₇ are as defined above or terminal epoxides such as 1,2 epoxides of the formula: 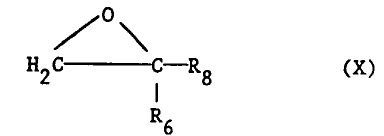 wherein R₆ and R₈ are as defined above. In both of the formulae IX and X, R₇ and R₆, or alternatively R₆ and R₈, may together with the interjacent carbon atom or atoms form a cycloalkylene epoxide or a vinylidene epoxide, preferably by forming a divalent linking hydrocarbyl group of 3 to 12 carbon atoms.

When R₆, R₇ and/or R₈ are oxy-substituted hydrocarbyl, preferred examples of compounds of formulae IX and X include nonylphenyl glycidyl ether, phenyl glycidyl ether, cresyl glycidyl ether, butyl glycidyl ether, alkyl C₁₂-C₁₃ glycidyl ether, alkyl C₈-C₁₀ glycidyl ether, 2-ethylhexyl glycidyl ether and isopropyl glycidyl ether.

In a particularly preferred process embodiment, terminal epoxides represented by formula X are utilized to form amido derivatives. These terminal epoxides are more preferably 1,2-epoxyalkanes, for example 1,2-epoxyethane, 1,2-epoxypropane, 1,2-epoxybutane, 1,2-epoxydecane, 1,2-epoxydodecane, 1,2-epoxyhexadecane, 1,2-epoxyoctadecane and mixtures thereof.

In the preparation of formula I compounds, the one or more cyclic ether, preferably epoxy, compounds may conveniently be reacted with compound of formula XI in a ratio from about 7:1 to about 55:1 mol of cyclic ether, preferably epoxy, compound per mol of compound of formula XI, and preferably at a molar ratio from about 10:1 to about 30:1, with the most preferred molar ratio being about 20:1.

As indicated above the reaction of the compound of formula XI with the cyclic ether, preferably epoxy, compound may conveniently be carried out in the presence of a basic potassium compound, which acts as alkoxylation catalyst. Such catalysts are conventional and include, for example, potassium methoxide, potassium ethoxide, potassium hydroxide, potassium hydride and potassium-t-butoxide. Potassium hydroxide and potassium-t-butoxide are preferred catalysts. The catalysts may be used in a base stable solvent such as alcohol, ether or hydrocarbons. The catalysts may be employed in a wide variety of concentrations. For example, basic potassium compounds may conveniently be used in an amount from about 0.02% to about 5.0% of total weight of reaction mixture, preferably from about 0.1% to about 2.0% of total weight of reaction mixture, and most preferably about 0.2% of total weight of reaction mixture.

Reaction may conveniently be carried out in a conventional autoclave reactor equipped with heating and cooling means. The process may be practised batchwise, continuously or semicontinuously.

The manner in which the alkoxylation reaction is conducted is not critical to the invention. Illustratively, a compound of formula XI and a potassium compound may be mixed and heated under vacuum for a period of at least 30 minutes. The one or more cyclic ether, preferably epoxy, compounds may then be added to the resulting mixture, the reactor sealed and pressurized with nitrogen, and the mixture stirred while reaction temperature is gradually increased.

Reaction temperature may conveniently be from about 80°C to about 250°C, preferably from about 100°C to about 150°C, and even more preferably from about 120°C to about 140°C. Reaction time may be conveniently from about 2 to about 20 hours, although longer or shorter times may be employed.

Similar processes to those above are known and are described, for example in U.S. Patent No. 4,973,414, U.S. Patent No. 4,883,826, U.S. Patent No. 5,123,932 and U.S. Patent No. 4,612,335.

The product of formula I is normally liquid and is recovered by known techniques such as filtration and distillation. The product is used in its crude state or is purified, if desired, by known techniques such as aqueous extraction, solid absorption and/or vacuum distillation to remove any remaining impurities.

Other methods for making the compounds of formula I will be apparent to those skilled in the art. For example, compounds of formula I may be prepared by reacting a compound of formula XI as described hereinbefore with cyclic ethers other than epoxy compounds. In addition, catalysts other than basic alkali metal compounds, for example acidic catalysts, may be employed in the preparation of compounds of formula I.

### Fuel Compositions

Compounds of formula I are useful as additives in fuel compositions which are burned, or combusted, in internal combustion engines. The fuel compositions of the present invention comprise a major amount of a mixture of hydrocarbons in the gasoline boiling range and a minor amount of one or more of the compounds of formula I. The "minor amount" is preferably less than about 10% by weight of the total fuel composition, preferably less than about 1% by weight of the total fuel composition and more preferably less than about 0.1% by weight of the total fuel composition.

Suitable liquid hydrocarbon fuels of the gasoline boiling range are mixtures of hydrocarbons having a boiling range of from about 25°C to about 232°C, and comprise mixtures of saturated hydrocarbons, olefinic hydrocarbons and aromatic hydrocarbons. Preferred are gasoline mixtures having a saturated hydrocarbon content ranging from about 40% to about 80% by volume, an olefinic hydrocarbon content from 0% to about 30% by volume and an aromatic hydrocarbon content from about 10% to about 60% by volume. The base fuel is derived from straight run gasoline, polymer gasoline, natural gasoline, dimer and trimerized olefins, synthetically produced aromatic hydrocarbon mixtures, from thermally or catalytically reformed hydrocarbons, or from catalytically cracked or thermally cracked petroleum stocks, and mixtures of these. The hydrocarbon composition and octane level of the base fuel are not critical. The octane level, (R+M)/2, will generally be above about 85.

Any conventional motor fuel base can be employed in the practice of the present invention. For example, hydrocarbons in the gasoline can be replaced by up to a substantial amount of conventional alcohols or ethers, conventionally known for use in fuels. The base fuels are desirably substantially free of water since water could impede a smooth combustion.

Normally, the hydrocarbon fuel mixtures to which the invention is applied may be leaded or unleaded, although are preferably substantially lead-free, and may contain minor amounts of blending agents such as methanol, ethanol, ethyl tertiary butyl ether, methyl tertiary butyl ether, and the like, at from about 0.1% by volume to about 15% by volume of the base fuel, although larger amounts may be utilized. The fuels can also contain conventional additives including antioxidants such as phenolics, e.g., 2,6-di-tert-butylphenol or phenylenediamines, e.g., N,N'-di-sec-butyl-p-phenylenediamine, dyes, metal deactivators, dehazers such as polyester-type ethoxylated alkylphenolformaldehyde resins. Corrosion inhibitors, such as a polyhydric alcohol ester of a succinic acid derivative having on at least one of its alpha-carbon atoms an unsubstituted or substituted aliphatic hydrocarbon group having from 20 to 500 carbon atoms, for example, pentaerythritol diester of polyisobutylene-substituted succinic acid, the polyisobutylene group having an average molecular weight of about 950, in an amount from about 1 ppm by weight to about 1000 ppm by weight, may also be present. The fuels can also contain antiknock compounds such as methyl cyclopentadienylmanganese tricarbonyl, tetraethyl lead or other lead-containing compounds, and ortho-azidophenol as well as co-antiknock compounds such as benzoyl acetone.

A preferred fuel composition of the invention thus additionally contains a minor amount of at least one additional additive compound selected from the group consisting of polyalkenyl amines, Mannich amines, polyalkenyl succinimide, poly(oxyalkylene)-carbamates, and poly(alkenyl)-N-substituted carbamates.

An effective amount of one or more compounds of formula I are introduced into the combustion zone of the engine in a variety of ways to prevent build-up of deposits, or to accomplish the reduction of intake valve deposits or the modification of existing deposits that are related to octane requirement. As mentioned, a preferred method is to add a minor amount of one or more compounds of formula I to the fuel. For example, one or more compounds of formula I are added directly to the fuel or are blended with one or more carriers and/or one or more hydrocarbon-soluble alkali metal or alkaline earth metal salts and/or one or more additional detergents before being added to the fuel.

The amount of cyclic amidoalcohol alkoxylates used will depend on the particular variation of formula I used, the engine, the fuel, and the presence or absence of carriers, additional detergents and diluents. Generally, each compound of formula I may conveniently be added in an amount up to about 1000 ppm by weight, especially from about 1 ppm by weight to about 600 ppm by weight based on the total weight of the fuel composition. Preferably, the amount will be at least 20 ppm and more preferably from about 50 ppm by weight to about 400 ppm by weight, and even more preferably from about 75 ppm by weight to about 250 ppm by weight based on the total weight of the fuel composition.

The carrier, when utilized, may conveniently have a weight average molecular weight from about 500 to about 5000. Suitable carriers, when utilized, include hydrocarbon based materials such as polyisobutylenes (PIB's), polypropylenes (PP's) and polyalphaolefins (PAO's), all of which may be hydrogenated or unhydrogenated but are preferably hydrogenated; polyether based materials such as polybutylene oxides (poly BO's), polypropylene oxides (poly PO's), polyhexadecene oxides (poly HO's) and mixtures thereof (i.e., both (poly BO) + (poly PO) and (poly-BO-PO)); and mineral oils such as those sold by member companies of the Royal Dutch/Shell group under the designations "HVI" and XHVI" (trade mark), Exxon Naphthenic 900 sus mineral oil and high viscosity index oils in general. The carrier is preferably selected from PIB's, poly BO's, and poly PO's, with poly BO's being the most preferred.

A particularly prepared carrier fluid comprises a combination of a polyalphaolefin having a viscosity at 100°C in the range 2 x 10⁻⁶ to 2 x 10⁻⁵ m²/s (2 to 20 centistokes), being a hydrogenated oligomer containing 18 to 80 carbon atoms derived from at least one alphaolefinic monomer containing from 8 to 16 carbon atoms, and a polyoxyalkylene compound selected from glycols, mono-and diethers thereof, having number average molecular weight (Mₙ) in the range 400 to 3000, the weight ratio polyalphaolefin: polyoxyalkylene compound being in the range 1:10 to 10:1.

The polyalphaolefins are primarily trimers, tetramers and pentamers, and synthesis of such materials is outlined in Campen et al., "Growing use of synlubes", Hydrocarbon Processing February 1982, Pages 75 to 82. The polyalphaolefin is preferably derived from an alphaolefinic monomer containing from 8 to 12 carbon atoms. Polyalphaolefins derived from decene-l have been found to be very effective. The polyalphaolefin preferably has viscosity at 100°C in the range of 6 x 10⁻⁶ to 1 x 10⁻⁵ m²/s (6 to 10 centistokes). Polyalphaolefin having a viscosity at 100°C of 8 x 10⁻⁶ m²/s (8 centistokes) has been found to be very effective.

Preferred polyoxyalkylene compounds for use in combination with these polyalphaolefins are described in EP-A-588429 (Applicants reference T 5677).

The carrier concentration in the final fuel composition is up to about 1000 ppm by weight. When a carrier is present, the preferred concentration is from about 50 ppm by weight to about 400 ppm by weight, based on the total weight of the fuel composition. Once the carrier is blended with one or more compounds of formula I, the blend is added directly to the fuel or packaged for future use.

The hydrocarbon-soluble alkali metal or alkaline earth metal salt, when utilised, may be one of those described in WO 87/01126, and the compounds of formula I are particularly suitable for incorporation, as additional component, in fuel compositions as described in WO 87/01126. Preferred hydrocarbon-soluble alkali metal or alkaline earth metal salts are, however, alkali metal or alkaline earth metal salts of a succinic acid derivative. Such a salt of a succinic acid derivative, when utilized, will have as a substituent on one of its alpha-carbon atoms and unsubstituted or substituted aliphatic hydrocarbon group having from 20 to 200 carbon atoms. Alternatively the succinic acid derivative will have as a substituent on one of its alpha-carbon atoms an unsubstituted or substituted hydrocarbon group having from 20 to 200 carbon atoms which is connected to the other alpha-carbon atom by means of a hydrocarbon moiety having from 1 to 6 carbon atoms, forming a ring structure. Suitable such salts are described for example in EP-A-207560 and in EP-A-491439.

The salts of the succinic acid derivative can be monobasic or dibasic. Monobasic salts in which the remaining carboxylic acid group has been transformed into an amide or ester group may also be used. Suitable alkali metal salts of a partial ester of an alkyl polyether alcohol with a succinic acid derivative are described in EP-A-491439.

Suitable metal salts include lithium, sodium, potassium, rubidium, cesium, and calcium salts. Particularly preferred salts are described in EP-A-207560.

The aliphatic hydrocarbon substituent(s) of the succinic acid derivative is suitably derived from a polyolefin, the monomers of which have 2 to 6 carbon atoms. Thus, convenient substituents include polyethylene, polypropylene, polybutylenes, polypentenes, polyhexenes or mixed polymers. Particularly preferred is an aliphatic hydrocarbon group which is derived from polyisobutylene.

The hydrocarbon group may include an alkyl and/or an alkenyl moiety, and may contain substituents. One or more hydrogen atoms may be replaced by another atom, for example halogen, or by a non-aliphatic organic group, e.g. an (un)substituted phenyl group, a hydroxy, ether, ketone, aldehyde or ester. A very suitable substituent in the hydrocarbon group is at least one other metal succinate group, yielding a hydrocarbon group having two or more succinate moieties.

The aliphatic hydrocarbon group should contain 20 to 200, preferably 35-150, carbon atoms. When a polyolefin is used as substituent the chain length is conveniently expressed as the number average molecular weight. The number average molecular weight of the substituent, e.g. determined by osmometry, is advantageously from 400 to 2000.

The succinic acid derivative may have more than one C₂₀₋₂₀₀ aliphatic hydrocarbon group attached to one or both alpha-carbon atoms, but preferably it has one C₂₀₋₂₀₀ aliphatic hydrocarbon group on one of its alpha-carbon atoms and on the other alpha-carbon atom either no substituent or a hydrocarbon of only a short chain length, e.g. C₁₋₆ group. The latter group can be linked with the C₂₀₋₂₀₀ hydrocarbon group forming a ring structure.

The fuel compositions of the present invention may also contain one or more additional detergents. When additional detergents are utilized, the fuel composition will comprise a mixture of a major amount of hydrocarbons in the gasoline boiling range as described hereinbefore, a minor amount of one or more compounds of formula I as described hereinbefore and a minor amount of an additional detergent selected from polyalkenyl amines, e.g. polybutyleneamines, such as "KEROCOM" polyisobutyleneamine, available ex BASF, Mannich amines, polyalkenyl succinimides, poly(oxyalkylene) carbamates, poly(alkenyl)-N-substituted carbamates, low molecular weight amines, e.g. C₁₀₋₂₀ alkylamines such as dodecylamine, and mixtures thereof. As noted above, a carrier as described hereinbefore may also be included. The "minor amount" is preferably less than about 10% by weight of the total fuel composition, more preferably less than about 1% by weight of the total fuel composition and yet more preferably less than about 0.1% by weight of the total fuel composition.

The polyalkenyl amine detergents utilized comprise at least one monovalent hydrocarbon group having at least 50 carbon atoms and at least one monovalent hydrocarbon group having at most five carbon atoms bound directly to separate nitrogen atoms of a diamine. Preferred polyalkenyl amines are polyisobutenyl amines. Polyisobutenyl amines are known in the art and representative examples are disclosed in various U.S. Patents including U.S. Patent No. 3,753,670, U.S. Patent No. 3,756,793, U.S. Patent No. 3,574,576 and U.S. Patent No. 3,438,757. Particularly preferred polyisobutenyl amines for use in the present fuel composition include N-polyisobutenyl-N',N'-dimethyl-1,3-diaminopropane (PIB-DAP), OGA-472 (a polyisobutenyl ethylene diamine available commercially from Oronite), N-polyisobutenyl diethylene triamine (PIB-DETA) and N-polyisobutenyl triethylene tetramine (PIB-TETA).

The Mannich amine detergents utilized comprise a condensation product of a high molecular weight alkyl-substituted hydroxyaromatic compound, an amine which contains an amino group having at least one active hydrogen atom (preferably a polyamine), and an aldehyde. Such Mannich amines are known in the art and are disclosed in U.S. Patent No. 4,231,759. Preferably, the Mannich amine is an alkyl substituted Mannich amine.

The polyalkenyl succinimide detergents comprise the reaction product of a dibasic acid anhydride with either a polyoxyalkylene diamine, a hydrocarbyl polyamine or mixtures of both. Typically the succinimide is substituted with the polyalkenyl group but the polyalkenyl group may be found on the polyoxyalkylene diamine or the hydrocarbyl polyamine. Polyalkenyl succinimides are also known in the art and representative examples are disclosed in various patent references including U.S. Patent No. 3,443,918, EP-A-208560, DE-OLS 3,126,404, U.S. Patent No. 4,234,435, U.S. Patent No. 4,810,261, U.S. Patent No. 4,852,993, U.S. Patent No. 4,968,321, U.S. Patent No.4,985,047, U.S. Patent No. 5,061,291 and U.S. Patent No. 5,147,414.

Particularly effective succinimide detergents are those obtained by reacting at least one amine, with a polyalkenyl derivative of a monoethylenically unsaturated C₄₋₁₀ dicarboxylic acid material in which the ratio of dicarboxylic acid moieties per polyalkenyl chain is not greater than 1.2:1 and the number average molecular weight (Mn) of the polyalkenyl chain is in the range from 1600 to 5000, e.g. as described in EP-A-587250 (Applicants reference T 1665).

Amines employed in the preparation of said succinimide detergents are preferably C₁₋₃₀, more preferably C₁₋₁₈, and especially C₈₋₁₂, amines containing 1 to 8 nitrogen atoms. Such amines may be branched or unbranched, saturated aliphatic, primary or secondary amines, containing 1 to 8 nitrogens, preferably mono-or diamines, such as ethylamine, butylamine, sec. butylamine, diethylamine and 3-dimethylamino-1-propylamine, but including higher polyamines such as alkylene polyamines, wherein pairs of nitrogen atoms are joined by alkylene groups of 2 to 4 carbon atoms.

The poly(oxyalkylene) carbamate detergents comprise an amine moiety and a poly(oxyalkylene) moiety linked together through a carbamate linkage, i.e.,

--O-C(O)-N-- (XI)

These poly(oxyalkylene) carbamates are known in the art and representative examples are disclosed for example in U.S. Patent No. 4,191,537, U.S. Patent No. 4,160,648, U.S. Patent No. 4,236,020, U.S. Patent No. 4,270,930, U.S. Patent No. 4,288,612 and U.S. Patent No. 4,881,945. Particularly preferred poly(oxyalkylene) carbamates for use in the present fuel composition include OGA-480 (a poly(oxyalkylene) carbamate which is available commercially from Oronite).

The poly(alkenyl)-N-substituted carbamate detergents utilized are of the formula: 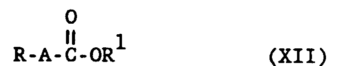 in which R is a poly(alkenyl) chain; R¹ is a hydrocarbyl or substituted hydrocarbyl group; and A is an N-substituted amino group. Poly(alkenyl)-N-substituted carbamates are known in the art and are disclosed in U.S. Patent No. 4,936,868.

Preferred low molecular weight amines are C₁₀₋₂₀ alkylamines. Aliphatic primary monoamines, particularly linear aliphatic primary monoamines, having 10 to 20 carbon atoms are particularly preferred. The alkylamine preferably has 10 to 18, e.g. 12 to 18, more preferably 12 to 16 carbon atoms. Dodecylamine is particularly preferred.

The one or more additional detergents are added directly to the hydrocarbons, blended with one or more carriers, blended with one or more compounds of Formula I, or blended with one or more compounds of Formula I and one or more carriers before being added to the hydrocarbons.

The concentration of the one or more additional detergents in the final fuel composition is generally up to about 1000 ppm by weight for each additional detergent. When one or more additional detergents are utilized, the preferred concentration for each additional detergent is from about 50 ppm by weight to about 400 ppm by weight, based on the total weight of the fuel composition, even more preferably from about 75 ppm by weight to about 250 ppm by weight, based on the total weight of the fuel composition.

Additive components can be added separately to the gasoline or can be blended with one or more diluents, forming an additive concentrate, and added to the gasoline together. Suitable gasoline-compatible diluents are hydrocarbons and mixtures of hydrocarbons with alcohols or ethers, such as methanol, ethanol, propanol, 2-butoxyethanol, methyl tert-butyl ether, or higher alcohols such as "Dobanol 91", (trade mark) available from member companies of the Royal Dutch/Shell group.

Preferably the diluent is an aromatic hydrocarbon solvent such as toluene, xylene, mixtures thereof or mixtures of toluene or xylene with an alcohol. Additionally preferred diluents include "Shellsol AB", "Shellsol R", (trade marks) and low aromatic white spirit (LAWS), which are available from member companies of the Royal Dutch/Shell group.

The invention further comprises an additive concentrate suitable for addition to gasoline which comprises a gasoline-compatible diluent and a compound of formula I as defined above, and optionally also at least one additional additive compound as defined above.

### Engine Tests

### Decreasing Intake Valve Deposits

The invention further provides a process for decreasing intake valve deposits in engines utilizing the fuel compositions of the present invention. The process comprises supplying to and combusting, or burning, in an internal combustion engine a fuel composition comprising a major amount of hydrocarbons in the gasoline boiling range and a minor amount of one or more compounds of formula I as defined above.

By supplying to and combusting or burning the fuel composition in an internal combustion engine, deposits in the induction system, particularly deposits on the tulips of the intake valves, are reduced. The reduction is determined by running an engine with clean induction system components and pre-weighed intake valves on dynamometer test stands in such a way as to simulate road operation using a variety of cycles at varying speeds while carefully controlling specific operating parameters. The tests are run for a specific period of time on the fuel composition to be tested. Upon completion of the test, the induction system deposits are visually rated, the valves are reweighed and the weight of the valve deposits is determined.

### Controlling Octane Requirement Increases

The invention further provides a process for controlling octane requirement increases in engines utilizing the fuel compositions of the present invention. The process comprises supplying to and combusting, or burning, in an internal combustion engine a fuel composition comprising a major amount of hydrocarbons in the gasoline boiling range and a minor amount of one or more compounds of formula I as defined above.

Octane requirement is the maximum octane number of a gasoline that presents trace knock in a given engine within the engine's normal operating range. An increase in octane requirement is generally experienced during mileage accumulation on a new engine. The increase is typically attributed to an increase in engine deposits. Octane requirement increase control is a performance feature that is usually expressed as a comparison of the octane requirement increase developed with a gasoline containing additives (test gasoline) relative to a version of the same gasoline without additives (base gasoline), i.e., the positive difference obtained by subtracting the results of gasoline containing additives from gasoline which does not contain additives.

The test protocol for octane requirement increase control must establish the stable octane requirement of the base gasoline relative to a clean engine. Base gasoline is typically the test gasoline without additives or special treatment; however, it may be gasoline containing additives for a specific comparison.

Octane requirement increase control testing consists of operating an engine assembled with clean combustion chambers and induction system components on a test gasoline to octane stabilization, measuring the octane requirement at regular intervals. The octane requirement increase control is the difference between the stabilized octane requirement of the engine operated on test gasoline and that of the stabilized octane requirement of the engine on base gasoline.

### Reduction of Octane Requirement

The invention still further provides a process for reducing octane requirement in engines utilizing the fuel compositions of the present invention. The process comprises supplying to and combusting, or burning, in an internal combustion engine a fuel composition comprising a major amount of hydrocarbons in the gasoline boiling range and a minor amount of one or more compounds of formula I as defined above.

Octane requirement reduction is the reduction of the octane requirement of an engine by the action of a particular gasoline, usually measured as a decrease from a stabilized octane requirement condition.

Octane requirement reduction is a performance feature that demonstrates a reduction from the established octane requirement of a base gasoline in a given engine. Octane requirement reduction testing consists of operating an engine, which has achieved stable octane requirement using base gasoline, on a test gasoline for approximately 100 hours. Octane measurements are made daily and octane requirement reduction is a reduction of octane requirement from that of base gasoline. Several octane requirement reduction tests may be conducted in a series for fuel to fuel comparison, or test fuel to base fuel comparison, by restabilizing on base fuel between octane requirement reduction tests.

The contribution of specific deposits is determined by removing deposits of interest and remeasuring octane requirement immediately after the engine is warmed to operating temperature. The octane requirement contribution of the deposit is the difference in ratings before and after deposit removal.

Compounds of formula I have been found to decompose during combustion to environmentally acceptable products, to produce very little residue and to be miscible with carriers and additional detergents.

The invention will be further understood from the following illustrative examples.

### Examples

### Compound Preparation

The cyclic amide alkoxylates used in the following examples were prepared by reacting an initiator with one or more epoxides in the presence of a potassium compound to produce compounds of formula I having a weight average molecular weight from about 600 to about 4000 as measured by gel permeation chromatography (GPC). In this specification, unless the context specifies otherwise, the term molecular weight and designation "Mw" refer to weight average molecular weight as measured by gel permeation chromatography (GPC), and estimated molecular weight is estimated relative molecular mass (average formula weight).

### Example 1

A mixture of N-(2-hydroxyethyl)-pyrrolidinone (52 g, 0.40 moles), potassium hydroxide (1.9 g in 1.5 g water) and toluene (200 g) was heated to 80°C under reduced pressure of 10 mm Hg (1.33 x 10³ Pa) for over 30 minutes. The mixture was added along with 1,2-epoxybutane (598 g, 8.3 moles) to a one litre autoclave equipped with heating and cooling means. The autoclave was sealed and purged of air by pressurizing and depressurizing with nitrogen at 50 psi (345 x 10³ Pa) several times. With stirring and an initial nitrogen pressure of 50 psi (345 x 10³ Pa), the mixture was heated slowly to 120°C and held at this temperature for over 12 hours. During the process, a maximum autogenous pressure at 125 psi (861 x 10³ Pa) at 127°C was observed. The pressure had dropped to 63 psi (434 x 10³ Pa) by the end of the reaction. The mixture was then cooled to ambient temperature, excess gas was vented and the product was recovered as a light brown liquid. This crude product was evaporated in a rotary evaporator at 80°C and 10 mm Hg (1.33 x 10³ Pa) to remove light material. Approximately 1.0 g of 1,2-epoxybutane was removed, indicating nearly quantitative conversion of the 1,2-epoxybutane.

GPC, NMR and IR analysis were consistent with the desired structure of a N-(2-hydroxyethyl)-pyrrolidinone butoxylate at approximately 20 units of 1,2-epoxybutane adduct.

The crude product was further purified using an extraction procedure to remove trace amounts of color body. The crude product was dissolved in hexane (50% basis of product) and extracted utilizing water three times at ambient temperature (20°C). The organic layer was then subjected to evaporation in a rotary evaporator at 80°C and 10 mm Hg (1.33 x 10³ Pa) to obtain a final product having an estimated molecular weight (average formula weight) of approximately 1600. GPC analysis indicated a weight average molecular weight of Mw=1250 and a polydispersity of 1.08.

### Example 2

The procedure of Example 1 was repeated with the following exceptions: a mixture of N-(2-hydroxyethyl)-pyrrolidinone (48g, 0.37 moles) and potassium hydroxide (1.7g in 1.7g water) was utilized in the absence of toluene. The mixture was added along with a mixture of 1,2-epoxybutane (442 g, 6.1 moles) and nonylphenol epoxide (110 g, 0.40 moles, an epoxy compound marketed as "HELOXY 64" (trade mark) by Rhone-Poulenc, with a structure derived from nonylphenol and epichlorohydrin) to the autoclave. The mixture was heated slowly to 1180C and maintained at this temperature for over 8 hours.

The product was confirmed by GPC, NMR and IR analysis, at approximately 17 units of 1,2-epoxybutane adduct and 1 unit of nonylphenol epoxide adduct. The crude product was further purified by the procedure of Example 1 to give a final product having an estimated molecular weight (average formula weight) of approximately 1600. GPC analysis indicated a weight average molecular weight of Mw-l100 and a polydispersity of 1.12.

### Example 3

The procedure of Example 1 was repeated with the following exceptions: a mixture of N-(2-hydroxyethyl)-pyrrolidinone (HEP) (43.3 g, 0.34 moles) and potassium hydroxide (1.7 g in 1.7 g water) was utilized in the absence of toluene. The resulting mixture was added along with propylene oxide (498 g, 8.6 moles) to the autoclave. The mixture was heated slowly to from 117°C to 120°C and maintained at this temperature for over 6 hours.

NMR analysis indicated a 71% yield of a HEP propoxylate having an average of 25 propylene oxide adducts. The crude product was further purified by the procedure of Example 1 to give a final product having an estimated molecular weight (average formula weight) of approximately 1600. GPC analysis indicated a weight average molecular weight of Mw-1260 and a polydispersity of 1.11.

### Example 4

The procedure of Example 1 was repeated with the exception that the crude product was further purified using a filtration procedure. The crude product was mixed with disodium dihydrogen pyrophosphate (Na₂H₂P₂O₇, 1.25 molar ratio to potassium) and water (5% wt basis of product). The mixture was heated to 80°C for over one hour with agitation. The slurry was filtered to remove solid material and to obtain a light-colored liquid as the final product containing less than 30 ppm by weight potassium and sodium.

### Example 5

The procedure of Example 1 was repeated with the following exceptions: a mixture of N-(2-hydroxyethyl)-pyrrolidinone (40 g, 0.31 moles) and potassium hydride (1.2 g) were mixed for over 30 minutes at ambient temperature (20°C). No toluene was used. The resulting liquid was added along with 1,2-epoxybutane (561 g, 7.8 moles) to the autoclave. The autoclave was sealed and purged of air and filled with nitrogen to 200 psi (1380 x 10³ Pa) while still at ambient temperature. The mixture was heated slowly to 137°C and maintained at this temperature for over 6 hours. During the process, a maximum autogenous pressure at 319 psi (2200 x 10³ Pa) at 137°C was observed. The pressure dropped to 271 psi (1870 x 10³ Pa) at the end of the reaction.

The crude product was purified using an extraction procedure in which the crude product was dissolved in hexane (50% basis of product) before being extracted three times with a mixture of methanol/ethanol/water at a ratio of approximately 1 : 0.2 : 1. The product was then subjected to evaporation in a rotary evaporator at 80°C and 10 mm Hg (1.33 x 10³ Pa) to obtain a light-colored (yellow) transparent fluid. A final product having an estimated molecular weight (average formula weight) of approximately 2000 was obtained, at approximately 26 units of 1,2-epoxybutane adduct. GPC analysis indicated a weight average molecular weight of Mw-1440 and a polydispersity of 1.09.

### Example 6

The procedure of Example 1 was repeated with the following exceptions: a mixture of N-(2-hydroxyethyl)-pyrrolidinone (59.5 g, 0.46 moles) and potassium hydroxide (1.7 g in 1.7 g water) was utilized in the absence of toluene. The resulting mixture was added along with 1,2-epoxybutane (540 g, 7.5 moles) to the autoclave. The mixture was heated slowly to 118°C and maintained at this temperature for over 7 hours. During the process, a maximum autogenous pressure at 125 psi (862 x 10³ Pa) at 120°C was observed.

The crude product was purified by the procedure of Example 1 to give a final product having an estimated molecular weight (average formula weight) of approximately 1300, at approximately 16 units of 1,2-epoxybutane adduct. GPC analysis indicated a weight average molecular weight of Mw-1060 and a polydispersity of 1.08.

### Example 7

The procedure of Example 1 was repeated with the following exception : a mixture of N-(2-hydroxyethyl)-pyrrolidinone (97 g, 0.75 moles), potassium hydroxide (1.4 g) and toluene (50 g) was stirred at ambient temperature (20°C) under nitrogen atmosphere until dissolved. The mixture was subjected to rotary evaporation under reduced pressure (10mm Hg) (1.33 x 10³ Pa) and heat (80°C) to remove water. The resulting liquid was added along with 1,2-epoxybutane (503 g, 7.0 moles) to the autoclave. The pressure was increased to 200 psi (1380 x 10³ Pa) and while stirring, the mixture was heated slowly to between 137°C and 140°C and held at this temperature for over 7 hours. During the process, a maximum autogenous pressure at 370 psi (2550 x 10³ Pa) was observed.

The crude product was purified by the procedure of Example 1 to give a final product having an estimated molecular weight (average formula weight) of approximately 800, at approximately 9 units of 1,2-epoxybutane adduct. GPC analysis indicated a weight average molecular weight of Mw=746 and a polydispersity of 1.05.

### Example 8

The procedure of Example 1 was repeated with the following exceptions: a mixture of 2-pyrrolidinone (32g, 0.38 moles) and potassium t-butoxide (1.7g) was utilized in the absence of toluene. The resulting material was added with 1,2-epoxybutane (568g, 7.9 moles) to the autoclave. The mixture was heated slowly to a temperature from 137°C to 141°C for over 7 hours. A pressure of from about 370 psi (2550 x 10³ Pa) to about 278 psi (1920 x 10³ Pa) was recorded during this time. The final product had an estimated molecular weight (average formula weight) of approximately 1600, at approximately 20 units of 1,2-epoxybutane adduct. GPC analysis indicated a weight average molecular weight of Mw=1240 and a polydispersity of 1.08.

### Example 9

The procedure of Example 1 was repeated with the following exceptions: a mixture of e-caprolactam (42.4g, 0.375 moles), potassium hydroxide (1.9g in 1.5 g water) and toluene (100g) was utilized. This mixture was added along with 1,2-epoxybutane (558g, 7.75 moles) to the autoclave. The mixture was slowly heated to from 118°C to 119°C under nitrogen pressure (73 psi (503 x 10³ Pa) to 123 psi (848 x 10³ Pa)) for over 6 hours. The crude product was purified by the procedure of Example 1 to give a final product having an estimated molecular weight (average formula weight) of approximately 1600, at approximately 20 units of 1,2-epoxybutane adduct. GPC analysis indicated an average molecular weight of Mw=1230 and a polydispersity of 1.09.

### Example 10

A mixture of N-(2-hydroxyethyl)-pyrrolidinone (HEP) (129 g, 1.0 moles) and potassium t-butoxide (0.5 g) was heated to approximately 80°C under a reduced pressure of 10 mm Hg (1.33 x 10³ Pa) for 30 minutes. The mixture was placed in a one litre autoclave, and the air was replaced with nitrogen. The reactor contents were pressured to 30 psi (207 x 10³ Pa) of nitrogen and heated to 150°C. Ethylene oxide (EO)(132 g, 3.0 moles) was fed slowly at 60 psi (414 x 10³ Pa) to the mixture over a 45 minute period. After the ethylene oxide was added, the mixture was maintained at 150°C for an additional 30 minutes to complete the reaction. The reaction was cooled to ambient temperature without neutralization and the ethoxylate adduct was isolated.

A portion of the ethoxylated adduct (52 g, 0.2 moles) was dissolved in butylene oxide (1,2-epoxybutane) (BO)(288 g, 4.0 moles) at 25°C and added to the autoclave. The autoclave was sealed, purged of air and pressurized to 50 psi (345 x 10³ Pa) using nitrogen. The system was heated to 140°C, producing a total pressure of 140 psi (965 x 10³) and maintained at this temperature for 5 hours. The reaction mixture was cooled to 25°C and the crude yellow product was evaporated in a rotary evaporator at 80°C and 10 mm Hg (1.33 x 10³ Pa) to remove any light (volatile) material. A total of 330 grams was isolated. The product was purified by aqueous extraction as described in Example 1. The product, containing 3 units of ethylene oxide adduct and 20 units of 1,2-epoxybutane adduct, could be represented by the schematic formula HEP(EO)₃(BO)₂₀H, and had an estimated molecular weight (average formula weight) of approximately 1800.

### Example 11

An ethoxylate adduct was produced according to the procedure of Example 10 using N-(2-hydroxyethyl)-pyrrolidinone (103.2 g, 0.8 moles), potassium-t-butoxide (0.7 g) and ethylene oxide (246.4 g, 5.6 moles). The product was isolated unneutralized.

A portion of this ethoxylated adduct (87.4 g, 0.2 moles) was reacted with butylene oxide (245 g, 3.4 moles) according to the procedure of Example 10. The product was isolated and purified by aqueous extraction as described in Example 1. The product, containing 7 units of ethylene oxide adduct and 17 units of 1,2-epoxybutane adduct, could be represented by the schematic formula HEP(EO)₇(BO)₁₇H and had an estimated molecular weight (average formula weight) of approximately 1800.

### Example 12

A mixture of N-(2-hydroxyethyl)-pyrrolidinone (23.2 g, 0.18 moles), potassium butoxide (1.0 g) and propylene oxide (301 g, 5.19 moles) was placed in an autoclave and pressurized to 200 psi (1380 x 10³ Pa) using nitrogen. The contents were allowed to react at 120°C for 9 hours. The pressure decreased from 395 psi (2720 x 10³ Pa) to 250 psi (1725 x10³ Pa) over the course of the reaction. The reaction mixture was cooled, and the yellow product purified by aqueous extraction as described in Example 1. The estimated molecular weight (average formula weight) of the product was approximately 1800, at approximately 29 units of propylene oxide adduct.

### Example 13

A mixture of N-(2-hydroxyethyl)-pyrrolidinone (23.2 g, 0.18 moles), potassium t-butoxide (1.0 g), propylene oxide (136 g, 2.34 moles) and 2-ethylhexyl glycidyl ether (obtained from Aldrich Chemical Company, 167 g, 0.9 moles) were reacted according to the procedure of Example 12. The final product was also purified by aqueous extraction as described in Example 1. The estimated molecular weight (average formula weight) of the product was approximately 1800, at approximately 14 units of propylene oxide adduct and 5 units of 2-ethylhexyl glycidyl ether adduct.

### Example 14

A mixture of N-(2-hydroxyethyl)-pyrrolidinone (21.9 g, 0.17 moles), potassium t-butoxide (1.0 g) and 2-ethylhexyl glycidyl ether (234 g, 1.53 moles) was reacted according to the procedure of Example 12. The final product was purified by aqueous extraction as described in Example 1 to obtain a product having an estimated molecular weight (average formula weight) of approximately 1800, at approximately 9 units of 2-ethylhexyl glycidyl ether adduct.

### Example 15

A mixture of N-(2-hydroxyethyl)-pyrrolidinone (21.9 g, 0.17 moles), potassium t-butoxide (1.0 g), butylene oxide (142 g, 1.97 moles) and 2-ethylhexyl glycidyl ether (142 g, 0.765 moles) were reacted according to the procedure of Example 12. The product was purified by aqueous extraction as described in Example 1 to obtain a product having an estimated molecular weight (average formula weight) of approximately 1800, at approximately 12 units of butylene oxide adduct and 5 units of 2-ethylhexyl glycidyl ether adduct.

### Example 16

A mixture of N-(2-hydroxyethyl)-pyrrolidinone (23.2 g, 0.18 moles), potassium t-butoxide (1.0 g) and isopropyl glycidyl ether (obtained from Aldrich Chemical Company, 301 g, 2.58 moles) was reacted according to the procedure of Example 12. The product was purified by aqueous extraction as described in Example 1 to obtain a product having an estimated molecular weight (average formula weight) of approximately 1800, at approximately 14 units of isopropyl glycidyl ether adduct.

### Example 17

600 g (0.375 moles) of the product of Example 1 was placed in a one litre, 3-necked round-bottomed flask equipped with a mechanical stirrer, temperature controller, Dean-stark trap and water condenser. Under nitrogen atmosphere, 2-ethylhexanoic acid (54 g, 0.375 moles) was added. While stirring under nitrogen flow, the mixture was heated to from about 130°C to about 180°C for over 10 hours. Some light boiling materials (13 ml) were removed through the trap. The resulting material (direct esterification product), having an estimated molecular weight (average formula weight) of approximately 1750, was further purified by extraction with water and evaporated in a rotary evaporator under 80°C, 10 mm Hg vacuum (1.33 x 10³ Pa). GPC analysis indicated a similar weight average molecular weight and distribution to the compound of Example 1 but with a slightly broader polydispersity (1.12 vs. 1.09).

### Example 18

The procedure of Example 1 was repeated with the exception that 300g (4.16 moles) of 1,2-epoxybutane was utilized to obtain a product having an estimated molecular weight (average formula weight) of 1800, at approximately 24 units of 1,2-epoxybutane adduct.

### Test Results

In each of the following tests, the base fuel utilized comprised either premium unleaded gasoline (PU) (90+ octane, [R+M/2]) (R = research octane number; M = motor octane number) and/or regular unleaded gasoline (RU) (85-88 octane, [R+M/2]). Those skilled in the art will recognize that fuels containing heavy catalytically cracked stocks, such as most regular fuels, are typically more difficult to additize in order to control deposits and effectuate octane requirement reduction and octane requirement increase control. The cyclic amide alkoxylate compounds utilized were prepared as indicated by Example number and were used at the concentration indicated in ppm by weight. The tests employed are described below and the results of the various tests are set forth in the tables below.

### Intake Valve Deposit Tests

Engines from vehicles were installed in dynamometer cells in such a way as to simulate road operation using a cycle of idle, low speed and high speed components while carefully controlling specific operating parameters. Fuels with and without the compounds of Formula I were tested in a variety of engines having port fuel injection including, 2.3 L Ford, 2.3 L Oldsmobile (Olds), 3.1 L Chevrolet (Chev) and 2.7 L BMW engines to determine the effectiveness of the instant compounds in reducing intake valve deposits ("L" refers to litre). 0.359 L Honda generator engines equipped with carburettors were also utilized to determine the effectiveness of the instant compounds in reducing intake valve deposits.

Before each test, the engine was inspected, the induction system components were cleaned and new intake valves were weighed and installed. The oil was changed and new oil and fuel filters, gaskets and spark plugs were installed.

In all engines except the Honda, the tests were run in cycles consisting of idle, 35 mph (56 kph) and 65 mph (104 kph) for a period of 100 hours unless indicated otherwise. In the Honda engines, the tests were run in cycles consisting of a no load idle mode for one minute followed by a three minute mode with a load at 2200 rpm for a period of 40 hours unless indicated otherwise. At the end of each test, the intake valves were removed and weighed.

**Table 2**

| Intake Valve Deposits in Honda Generator Engines | | | |
|---|---|---|---|
| Compound | Conc. ppm | | Average Deposit |
| Example # | Fuel | by weight | Weight, mg |
| 1 | PU | 200 | 10.5* |
| - | PU | 0 | 25.1* |
| 1 | PU | 200 | 16,10 |
| 2 | PU | 200 | 5 |
| 3 | PU | 200 | 17 |
| 7 | PU | 200 | 23 |
| 9 | PU | 200 | 12 |
| - | PU | 0 | 39 |
| 6 | PU | 200 | 27 |
| - | PU | 0 | 83,79 |
| 1 | RU | 200 | 39 |
| 1 | RU | 300 | 34 |
| 2 | RU | 200 | 34 |
| - | RU | 0 | 98 |

| | | | |
|---|---|---|---|
| * Indicates an average of five tests in three different Honda generator engines. | | | |

Results of these tests demonstrate that the compounds of the present invention are very useful in significantly preventing the accumulation of deposits on the intake valves in the generator engines tested as compared to the effects of the base fuel (indicated by no compound example # and 0 ppm by weight) as shown by the much lower average valve deposits.

**TABLE 3**

| Intake Valve Deposits in Various Engines | | | | |
|---|---|---|---|---|
| Compound | | Fuel | Conc. ppm | Average Deposit |
| Example # | Engine | | by weight | Weight, mg |
| - | 2.8 L Chev | PU | 0 | 232* |
| 1 | 2.8 L Chev | PU | 200 | 212 |
| 1 | 2.8 L Chev | PU | 400 | 55 |
| - | 2.3 L Olds | PU | 0 | 174* |
| 1 | 2.3 L Olds | PU | 200 | 56 |
| 1 | 2.3 L Olds | PU | 300 | 67 |
| - | 3.1 L Chev | PU | 0 | 72 |
| 1 | 3.1 L Chev | PU | 200 | 38,69 |
| 9 | 3.1 L Chev | PU | 200 | 33 |
| - | 2.7 L BMW | PU | 0 | 138* |
| 1 | 2.7 L BMW | PU | 200 | 86 |
| 4 | 2.7 L BMW | PU | 200 | 123 |
| 5 | 2.7 L BMW | PU | 200 | 115 |
| - | 2.3 L Ford | PU | 0 | 242 |
| 1 | 2.3 L Ford | PU | 200 | 59 |
| 2 | 2.3 L Ford | PU | 200 | 122 |
| 9 | 2.3 L Ford | PU | 200 | 50 |

| | | | | |
|---|---|---|---|---|
| * Indicates that base fuel having a different blend but the same octane was utilized. | | | | |

The results given in Table 3 demonstrate that the compounds of the present invention were very useful in significantly preventing the accumulation of deposits on the intake valves in those tests as compared to the effects of the base fuel (indicated by no compound example # and 0 ppm by weight), as shown by the lower average valve deposits.

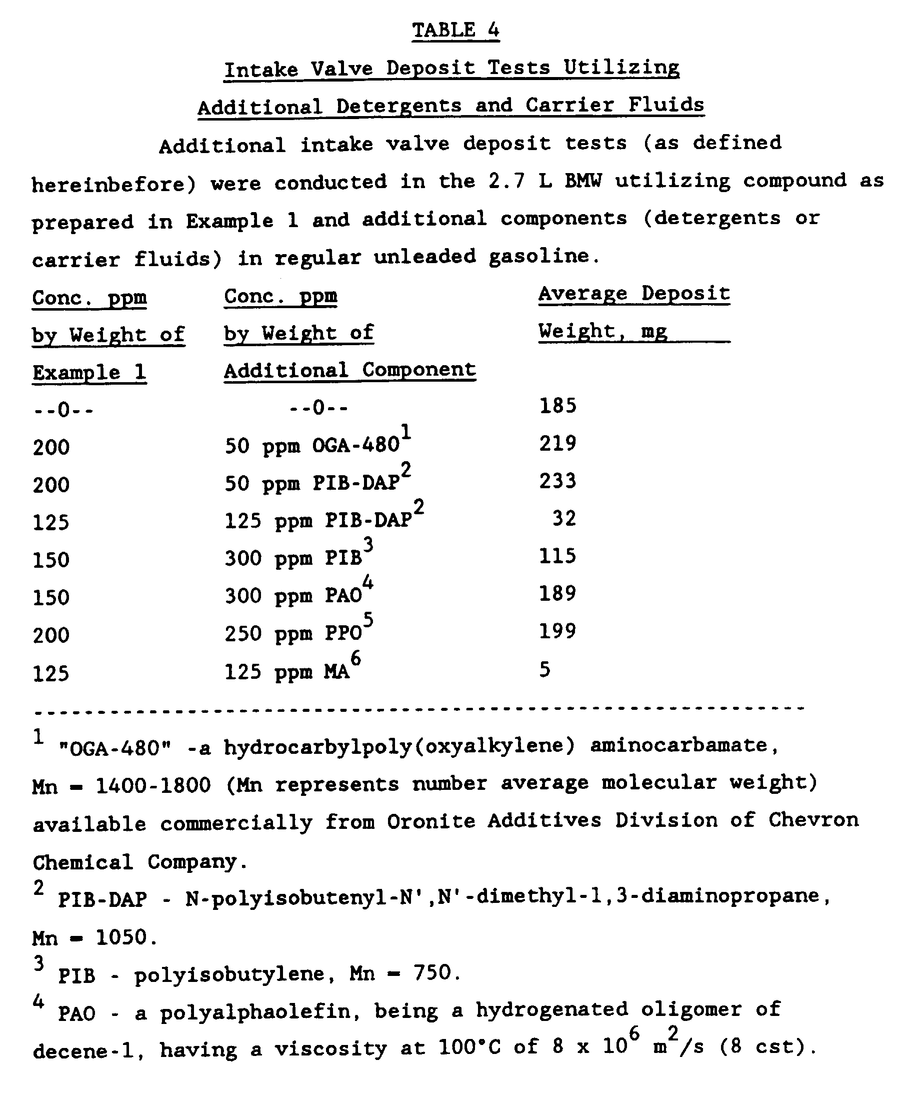

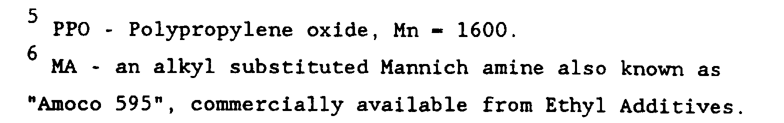

Results of these tests demonstrate that compounds of the present invention, when combined with carrier fluids or additional detergents are useful in preventing the accumulation of deposits on intake valves.

**TABLE 5**

| Intake Valve Deposit Tests Utilizing Additional Detergents and Carrier Fluids | | |
|---|---|---|
| Additional intake valve deposit tests (as defined herein before) were conducted in the 0.359 L Honda generator engine utilizing compounds as prepared in Example 1 and additional components (detergents or carrier fluids) in regular unleaded gasoline. | | |
| Conc. ppm by weight of Example 1 | Conc. ppm by weight of Additional Component | Average Deposit Weight, mg |
| 125 | --0-- | 49.0 |
| 125 | 125 ppm PIB-DAP¹ | 0.4 |
| 125 | 125 ppm MPDC² | 29.3 |
| 125 | 300 ppm EN-900³ | 29.4 |

| | | |
|---|---|---|
| ¹ PIB-DAP - N-polyisobutenyl-N',N'-dimethyl-1,3-diaminopropane, Mn = 1050. | | |
| ² MPDG - Methyl PIB-DAP Carbamate, Mn = 1100. | | |
| ³ EN-900 - Exxon Naphthenic 900 sus mineral oil. | | |

Results of these tests further demonstrate that the compounds of the present invention, when combined with carrier fluids or co-detergents are useful in preventing the accumulation of deposits on intake valves.

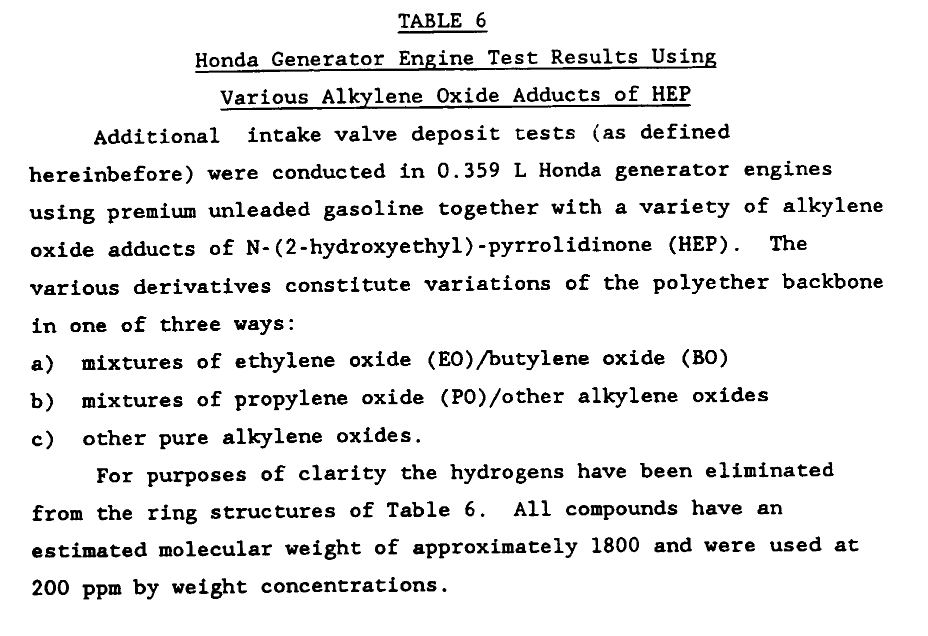

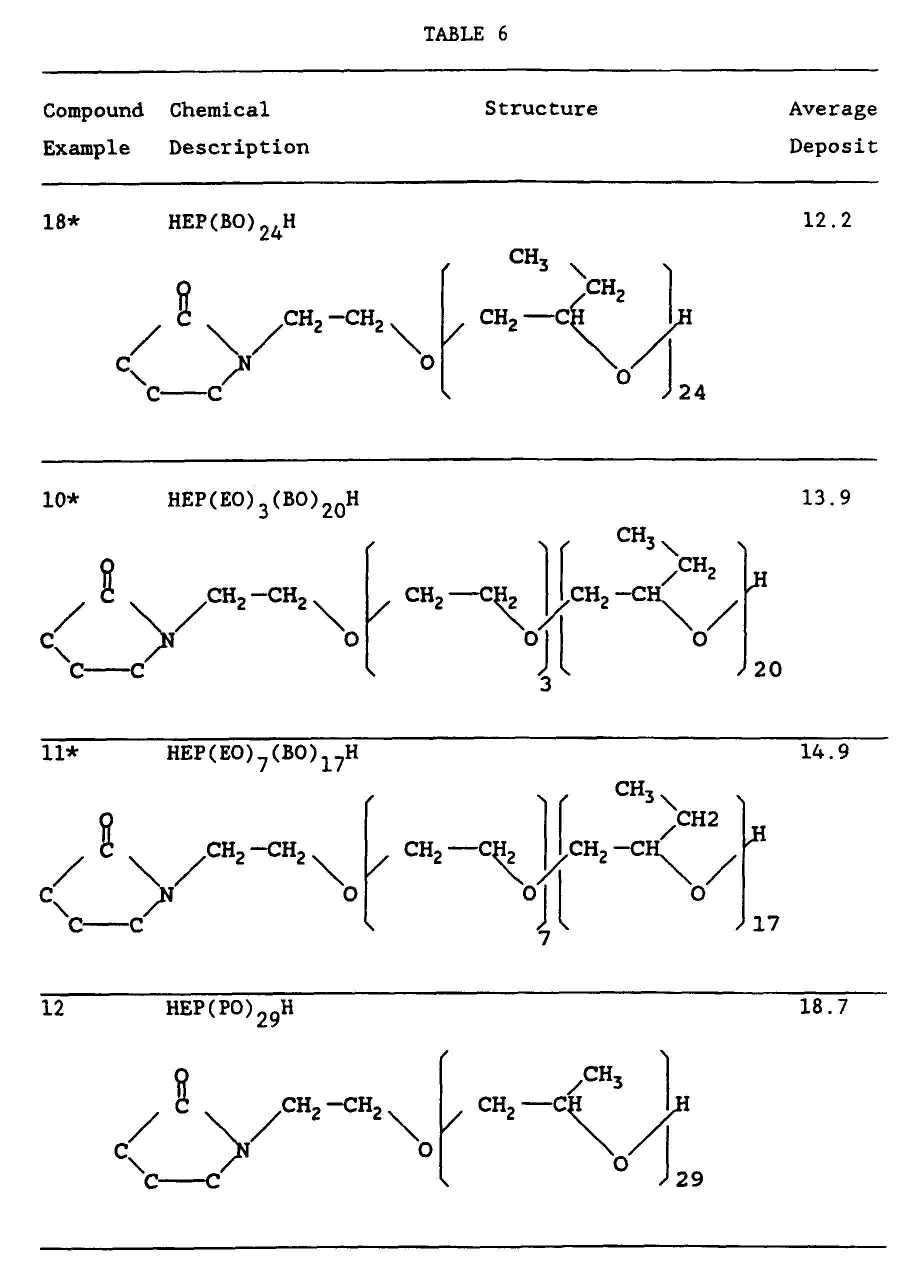

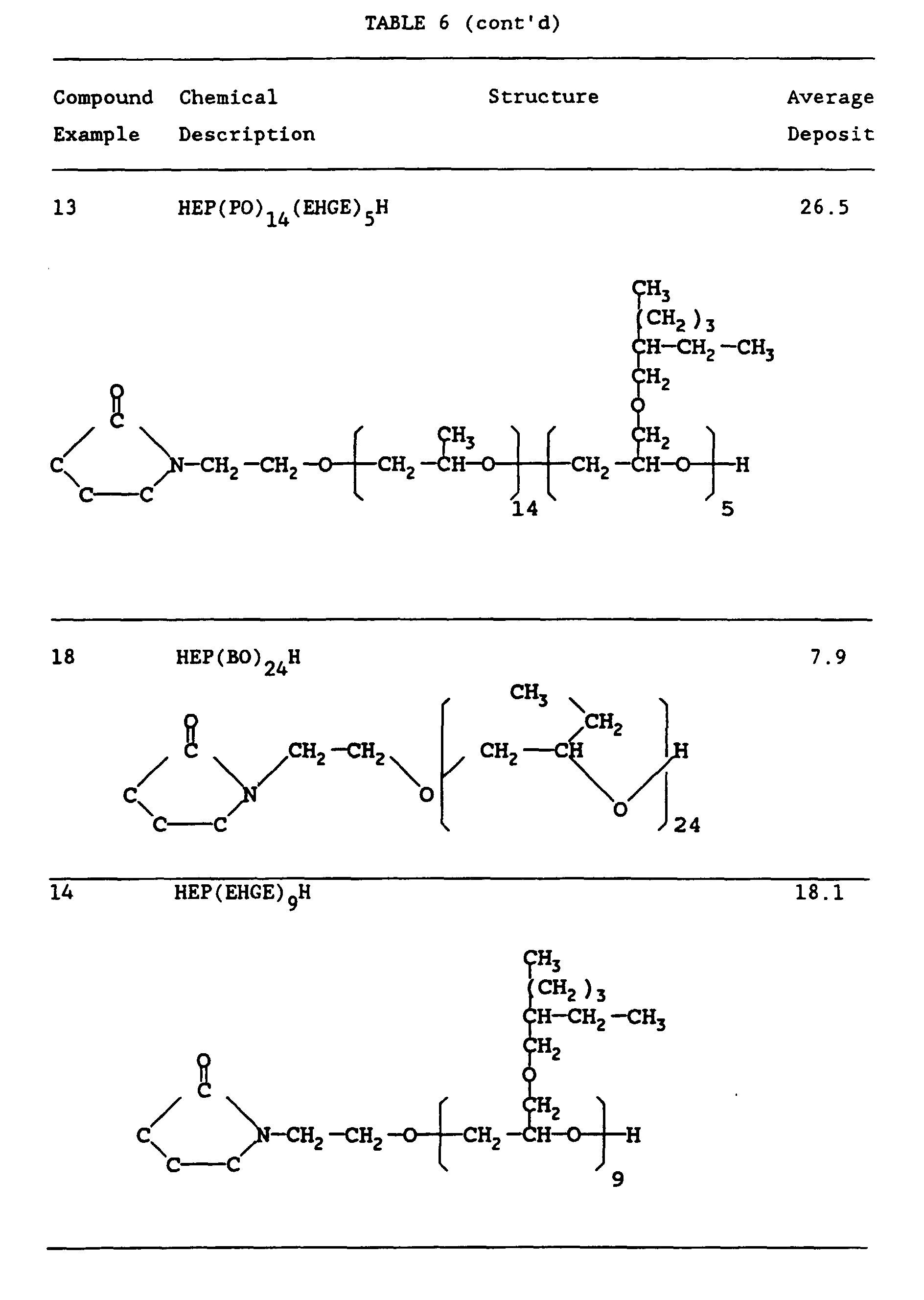

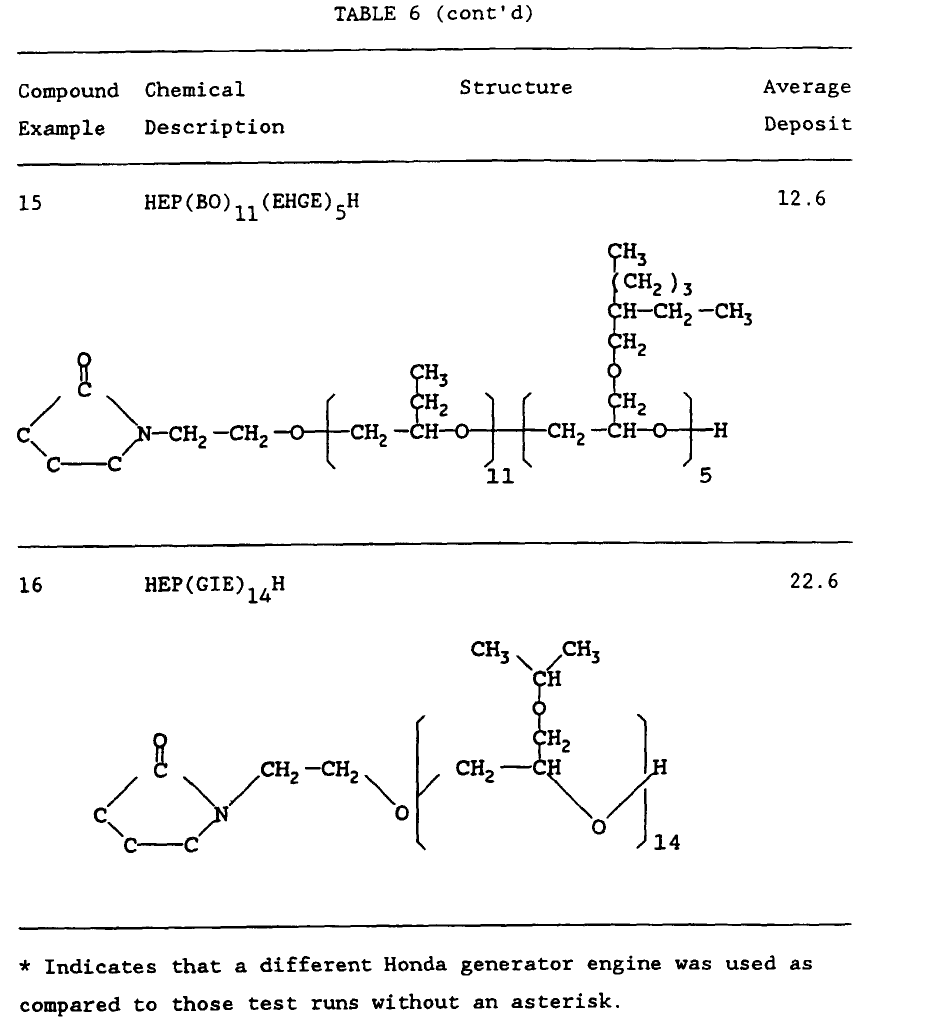

The following abbreviations were used throughout Table 6:
- HEP =: n-(2-hydroxyethyl)-pyrrolidinone
- BO =: butylene oxide
- EO =: ethylene oxide
- PO =: propylene oxide
- EHGE =: ethylhexyl glycidyl ether
- GIE =: glycidyl isopropyl ether

### Method For Octane Requirement Reduction and Octane Requirement Increase Control

The purpose of octane requirement tests in engine dynamometer cells is to provide a method of determining the effect of various gasoline components and additives upon the octane requirement of the engine. Measurement of the effect of the induction system and combustion chamber deposits on octane requirement may also be performed.

Engines from vehicles are installed in dynamometer cells in such a way as to simulate road operation using a cycle of idle, low speed and high speed components while carefully controlling specific operating parameters. Two types of octane requirement test are conducted: octane requirement increase control and octane requirement reduction. Contribution of specific deposits to octane requirement may also be determined.

Prior to testing, each engine is inspected and has its induction system cleaned. Parts are checked for excessive wear and a new oil filter, fuel filter, intake valves and spark plugs are installed.

Octane requirement is measured initially with the clean engine, then at specific intervals, until a stable requirement is established. Test stand engines reach an octane stabilization in about 250 hours, or 9500 miles (15,200 km) equivalent (168 hours per week). After stabilization, the engine is disassembled, cleaned, reassembled and the octane requirement measured again. This second clean engine octane requirement is referred to as "check back" since it checks back to the initial requirement. The check back octane requirement is the test reference, as it accommodates engine changes that occur throughout the test. A check back octane requirement significantly different from the initial requirement indicates a problem with the test. The difference between the check back octane requirement and the stable octane requirement is the octane requirement increase achieved during the test.

The entire process is repeated using the test gasoline. An octane requirement level established by the test gasoline less than the base gasoline represents octane requirement increase control favorable to the test gasoline.

Octane requirement reduction is a performance feature that demonstrates a reduction from the established octane requirement of a base gasoline in a given engine. The test need not start with a clean engine. The test protocol requires measurement of the octane requirement of an engine fuelled with a base gasoline which generally consists of the test gasoline without additives or special treatment. However, the base gasoline may contain additives for a specific comparison. After reaching a stable octane requirement with the base gasoline, the engine is operated on test gasoline until the octane requirement again stabilizes. Rating intervals for test stands are typically twenty-four hours. Test stand engines may be used to conduct several octane requirement reduction tests in sequence with the engine being restabilized on base gasoline between each test. A stable reduction of octane requirement from that of the base gasoline represents octane requirement reduction favourable to the test gasoline.

**TABLE 7**

| OCTANE REQUIREMENT INCREASE CONTROL TESTING | | |
|---|---|---|
| All tests were conducted using 200 ppm by weight of Example 1. | | |
| Test Engine | Fuel | Base Fuel Octane Requirement Minus Test Fuel Octane Requirement* |
| 1989 3.8 L Buick | PU | 5 |
| 1989 3.8 L Buick | RU | 1 |
| 1990 3.1 L Chev | PU | 3 |
| 1990 3.1 L Chev | RU | 1 |
| 1987 2.3 L Ford | PU | 3 |
| 1988 2.3 L Olds | PU | 2 |

| | | |
|---|---|---|
| *Positive numbers indicate good octane control performance. | | |

The overall results indicate that the compound of Example 1 possesses the ability to control octane requirement increases relative to base fuel.

**TABLE 8**

| OCTANE REQUIREMENT REDUCTION TESTING | | | |
|---|---|---|---|
| All tests were conducted using 200 ppm by weight of the compound indicated. | | | |
| Compound Example # | Test Engine | Fuel | Base Fuel Octane Requirement Minus Test Fuel Octane Requirement* |
| 1 | 1989 3.8 L Buick | PU | 2 |
| 1 | 1990 3.1 L Chev | PU | 4 |
| 1 | 1990 3.1 L Chev | PU plus Deposit Control Package** | 3 |
| 1 | 1990 3.1 L Chev | RU | 2 |
| 1 | 1987 2.3 L Ford | PU | 3 |
| 1 | 1988 2.3 L Olds | PU | 3 |
| 9 | 1990 3.1 L Chev | PU | 3 |

| | | | |
|---|---|---|---|
| *Positive numbers indicate good octane requirement reduction performance. | | | |
| **The base fuel contains an additive package comprising a PIB amine and a synthetic carrier for comparative purposes. | | | |

The results indicate that the compound of Example 1 possesses the ability to reduce octane requirement relative to base fuel. The engine test utilizing Example 9 also shows octane requirement reduction.

### BMW ROAD TESTS - INTAKE VALVE DEPOSITS

10,000 mile (16,000 km) and 5,000 mile (8,000 km) tests to determine intake valve deposits were conducted in 1985 BMW 318i cars having in-line, four cylinder, 4-stroke, water cooled gasoline engines that have a single overhead camshaft, two valves per cylinder and a displacement of 1.8 litres. The engines were port fuel injected equipped with Bosch port fuel-injectors and L-Jetronic fuel management systems. All cars used were equipped with overdrive automatic transmissions.

Before the test started, all combustion chamber deposits were removed from the engine head, intake manifold and piston tops. New intake valves were weighed and installed. The oil and filters were changed, new spark plugs were installed and the fuel injectors flow checked. Mileage was accumulated on public roads using trained drivers. The test route consisted of about 10% city driving (varied speeds with stop-and-go idling), 20% on secondary roads (moderate speeds with infrequent stops) and 70% highway driving (maximum speed of 65 mph).

The primary test data is the intake valve deposits weights at the end of a 10,000 mile (16,000 km) test. BMW's pass criteria are as follows: an average deposit weight of 100 milligrams/valve or less at the conclusion of the 10,000 mile (16,000 km) test meets BMW requirements for unlimited mileage acceptance; an average deposit weight of 250 mg/valve or less at the conclusion of the test meets BMW's requirement for 50,000 mile (80,000 km) service.

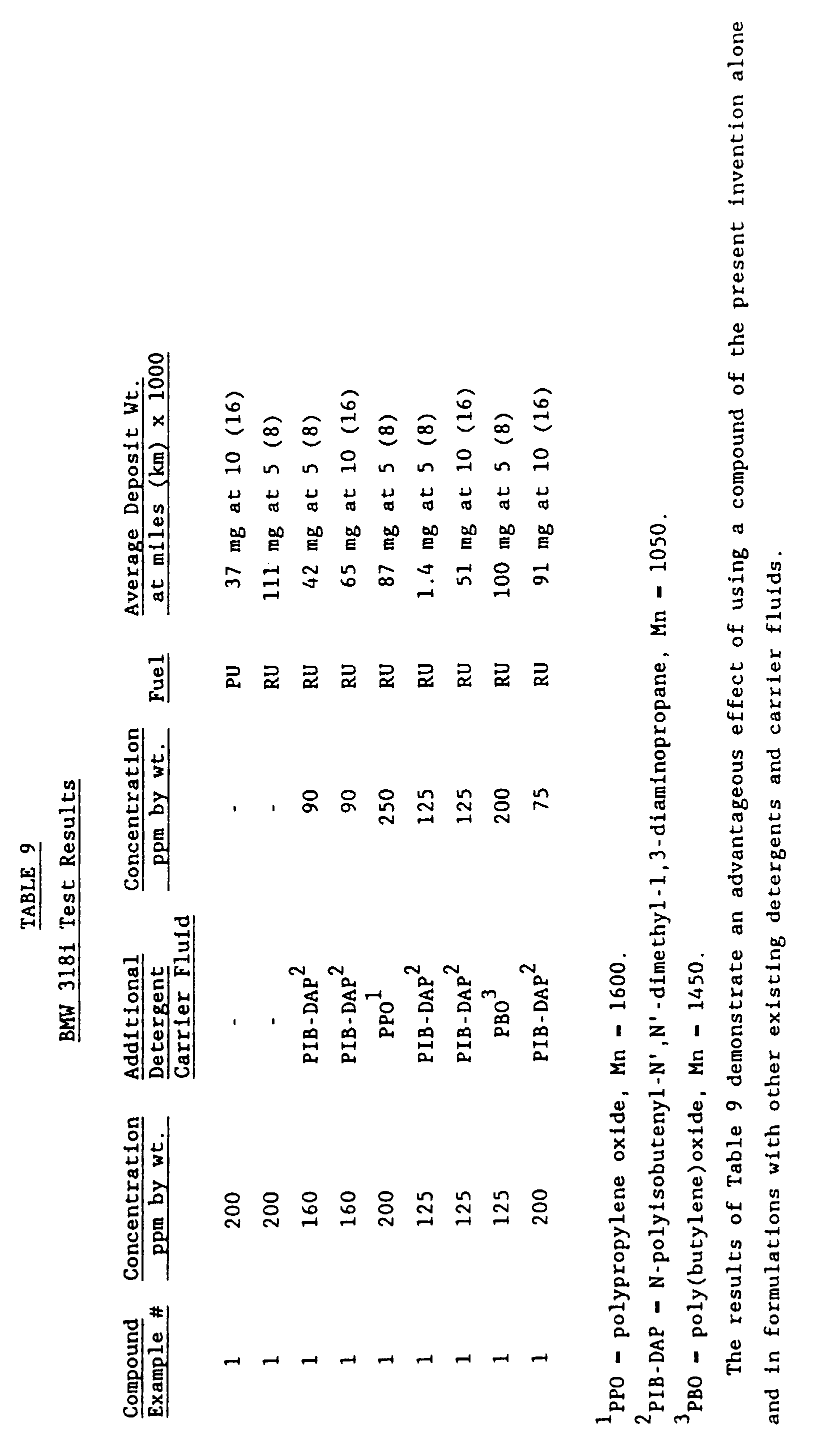

## Claims

1. A fuel composition comprising a mixture of a major amount of hydrocarbons in the gasoline boiling range and a minor amount of an additive compound having the general formula I: 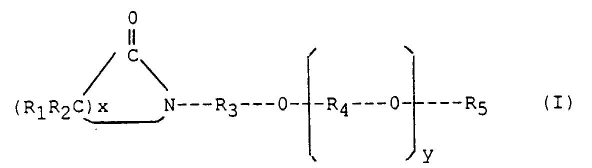 wherein x is from 2 to 20; y is from 1 to 50; R₁ and R₂ are independently hydrogen or optionally substituted hydrocarbyl of 1 to 100 carbon atoms; R₃ is optionally substituted hydrocarbyl of 1 to 100 carbon atoms; each R₄ is independently optionally substituted hydrocarbyl of 2 to 100 carbon atoms of the formula: 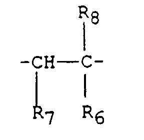 wherein R₆, R₇ and R₈ are each independently hydrogen or optionally substituted hydrocarbyl, provided that at least one of R₇ and R₈ is hydrogen, such that R₆ and R₈ together or R₆ and R₇ together contain 0 to 98 carbon atoms, or R₆ and R₈ or R₆ and R₇ together represent a hydrocarbyl bridge containing 3 to 98 carbon atoms; R₅ is hydrogen , or optionally substituted hydrocarbyl of 1 to 100 carbon atoms or acyl of 1 to 20 carbon atoms and the average formula weight of the additive compound is a least 600.

2. A fuel composition according to claim 1 wherein each of R₆, R₇ and R₈ is independently hydrogen or optionally substituted hydrocarbyl of 1 to 18 carbon atoms, provided that at least one of R₇ and R₈ is hydrogen.

3. A fuel composition according to claim 1 or 2 wherein the average formula weight of the additive compound is in the range from 800 to 4000.

4. A fuel composition according to any one of claims 1 to 3 wherein x is from 3 to 11.

5. A fuel composition according to any one of claims 1 to 4 wherein y is from 8 to 40.

6. A fuel composition according to any one of claims 1 to 5 wherein R₃ is hydrocarbyl of 1 to 20 carbon atoms.

7. A fuel composition according to any one of claims 1 to 6 wherein x is from 3 to 5, R₁ and R₂ are independently hydrogen or hydrocarbyl of 1 to 8 carbon atoms; R₃ is hydrocarbyl of 2 to 4 carbon atoms; R₅ is hydrogen; each R₆ is independently hydrogen, alkyl of 1 to 18 carbon atoms or oxy-substituted hydrocarbyl of 1 to 18 carbon atoms; and R₇ and R₈ are both hydrogen.

8. A fuel composition according to claim 7 wherein when one or more moieties R₆ are oxy-substituted hydrocarbyl said one or more moieties are independently selected from alkoxy-substituted alkylene of 1 to 18 carbon atoms or aryloxy-substituted alkylene of 1 to 18 carbon atoms.

9. A fuel composition according to claim 7 or 8 wherein when one or more moieties R₆ are an oxy-substituted hydrocarbyl, said one or more moieties are independently selected from ethylhexyleneoxymethylene, isopropoxymethylene, butoxymethylene, nonylphenoxymethylene and phenoxymethylene.

10. A fuel composition according to any one of claims 1 to 7 wherein x is 3 to 5; R₃ is hydrocarbyl of two to four carbon atoms; each R₆ is independently hydrogen or hydrocarbyl comprising alkyl of 1 to 2 carbon atoms; R₇ and R₈ are both hydrogen; and R₅ is hydrogen.

11. A fuel composition according to any one of claims 1 to 10 wherein R₁ and R₂ are each hydrogen.

12. A fuel composition according to any one of claims 1 to 11 wherein the additive compound is present in an amount from 50 ppm by weight to 400 ppm by weight based on the total weight of the fuel composition.

13. A fuel composition according to any one of claims 1 to 12 which additionally contains a minor amount of at least one additional additive compound selected from the group consisting of polyalkenyl amines, Mannich amines, polyalkenyl succinimides, poly(oxyalkylene)carbamates, and poly(alkenyl)-N-substituted carbamates.

14. A method of operating an internal combustion engine and controlling octane requirement increase and/or reducing octane requirement and/or reducing intake valve deposits, which comprises burning in said engine a fuel composition according to any one of claims 1 to 13.

15. An additive concentrate suitable for addition to gasoline which comprises a gasoline-compatible diluent and a compound of formula I as defined in any one of claims 1 to 11, and optionally also at least one additional additive compound as defined in claim 13.

16. A compound of formula I as defined in any one of claims 1 to 11 wherein R₃ or at least one R₄ moiety is optionally substituted hydrocarbyl of at least four carbon atoms.

17. A process for the preparation of a compound of formula I as defined in claim 16 which comprises reacting a compound of general formula XI 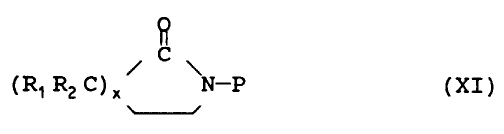 wherein x, R₁ and R₂ are as defined in claim 1 and P is hydrogen or a group of formula -R₃-OH, wherein R₃ is as defined in claim 1 with at least one cyclic ether compound which upon ring opening leaves a moiety -R₄-O-, provided that when P is hydrogen, the compound of formula XI is reacted with at least one mol of cyclic ether compound which leaves a moiety -R₃-O- upon ring opening per mol of compound of formula XI, to form a compound of formula I wherein R₅ is hydrogen, optionally followed by etherification or esterification to form a compound of formula I wherein R₅ is optionally substituted hydrocarbyl of 1 to 100 carbon atoms or acyl of 1 to 20 carbon atoms.

## Patentansprüche

1. Kraftstoffzusammensetzung, enthaltend ein Gemisch aus einer größeren Menge an Kohlenwasserstoffen, die im Benzinbereich sieden, und einer kleineren Menge eines Additivs mit der allgemeinen Formel I: 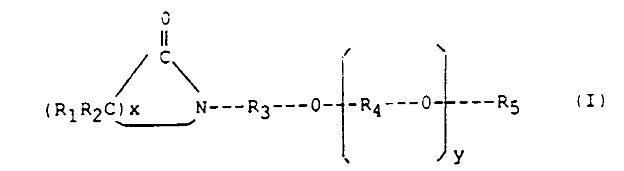 worin x 2 bis 20; y 1 bis 50; R₁ und R₂ unabhängig voneinander Wasserstoff oder einen gegebenenfalls substituierten Kohlenwasserstoffrest mit 1 bis 100 Kohlenstoffatomen; R₃ einen gegebenenfalls substituierten Kohlenwasserstoffrest mit 1 bis 100 Kohlenstoffatomen; R₄ jeweils unabhängig einen gegebenenfalls substituierten Kohlenwasserstoffrest mit 2 bis 100 Kohlenstoffatomen der Formel: 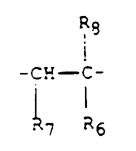 worin R₆, R₇ und R₈ jeweils unabhängig voneinander Wasserstoff oder einen gegebenenfalls substituierten Kohlenwasserstoffrest bedeuten, vorausgesetzt, daß mindestens einer der Reste R₇ und R₈ Wasserstoff bedeutet, so daß R₆ und R₈ zusammen oder R₆ und R₇ zusammen 0 bis 98 Kohlenstoffatome enthalten, oder R₆ und R₈ oder R₆ und R₇ zusammen für eine Kohlenwasserstoffbrücke mit 3 bis 98 Kohlenstoffatomen stehen; und R₅ Wasserstoff oder einen gegebenenfalls substituierten Kohlenwasserstoffrest mit 1 bis 100 Kohlenstoffatomen oder einen Acylrest mit 1 bis 20 Kohlenstoffatomen bedeuten und das mittlere Formelgewicht des Additivs mindestens 600 beträgt.

2. Kraftstoffzusanmensetzung nach Anspruch 1, wobei R₆, R₇ und R₈ jeweils unabhängig voneinander Wasserstoff oder einen gegebenenfalls substituierten Kohlenwasserstoffrest mit 1 bis 18 Kohlenstoffatomen bedeuten, vorausgesetzt, daß mindestens einer der Reste R₇ und R₈ Wasserstoff bedeutet.

3. Kraftstoffzusamwensetzung nach Anspruch 1 oder 2, wobei das mittlere Formelgewicht des Additivs im Bereich von 800 bis 4000 liegt.

4. Kraftstoffzusamwensetzung nach einem der Ansprüche 1 bis 3, wobei x 3 bis 11 bedeutet.

5. Kraftstoffzusanmensetzung nach einem der Ansprüche 1 bis 4, wobei y 8 bis 40 bedeutet.

6. Kraftstoffzusamwensetzung nach einem der Ansprüche 1 bis 5, wobei R₃ einen Kohlenwasserstoffrest mit 1 bis 20 Kohlenstoffatomen bedeutet.

7. Kraftstoffzusamwensetzung nach einem der Ansprüche 1 bis 6, wobei x 3 bis 5, R₁ und R₂ unabhängig voneinander Wasserstoff oder einen Kohlenwasserstoffrest mit 1 bis 8 Kohlenstoffatomen; R₃ einen Kohlenwasserstoffrest mit 2 bis 4 Kohlenstoffatomen; R₅ Wasserstoff; R₆ jeweils unabhängig Wasserstoff, einen Alkylrest mit 1 bis 18 Kohlenstoffatomen oder einen oxysubstituierten Kohlenwasserstoffrest mit 1 bis 18 Kohlenstoffatomen; und R₇ und R₈ beide Wasserstoff bedeuten.

8. Kraftstoffzusamwensetzung nach Anspruch 7, wobei in dem Fall, daß eine oder mehrere Gruppierungen R₆ einen oxysubstituierten Kohlenwasserstoffrest bedeuten, diese eine oder mehrere Gruppierungen unabhängig voneinander unter alkoxysubstituierten Alkylenresten mit 1 bis 18 Kohlenstoffatomen oder aryloxysubstituierten Alkylenresten mit 1 bis 18 Kohlenstoffatomen ausgewählt sind.

9. Kraftstoffzusamwensetzung nach Anspruch 7 oder 8, wobei in dem Fall, daß eine oder mehrere Gruppierungen R₆ einen oxysubstituierten Kohlenwasserstoffrest bedeuten, diese eine oder mehrere Gruppierungen unabhängig voneinander unter Ethylhexylenoxymethylen, Isopropoxymethylen, Butoxymethylen, Nonylphenoxymethylen und Phenoxymethylen ausgewählt sind.

10. Kraftstoffzusammensetzung nach einem der Ansprüche 1 bis 7, wobei x 3 bis 5; R₃ einen Kohlenwasserstoffrest mit zwei bis vier Kohlenstoffatomen; R₆ jeweils unabhängig Wasserstoff oder einen Kohlenwasserstoffrest, bestehend aus einem Alkylrest mit 1 bis 2 Kohlenstoffatomen; R₇ und R₈ beide Wasserstoff; und R₅ Wasserstoff bedeuten.

11. Kraftstoffzusammensetzung nach einem der Ansprüche 1 bis 10, wobei R₁ und R₂ jeweils Wasserstoff bedeuten.

12. Kraftstoffzusammensetzung nach einem der Ansprüche 1 bis 11, wobei das Additiv in einer Menge von 50 Gew.-ppm bis 400 Gew.-ppm, bezogen auf das Gesamtgewicht der Kraftstoffzusammensetzung, vorliegt.

13. Kraftstoffzusammensetzung nach einem der Ansprüche 1 bis 12, die außerdem eine kleinere Menge an mindestens einem weiteren Additiv enthält, das aus der Gruppe, bestehend aus Polyalkenylaminen, Mannich-Aminen, Polyalkenylsuccinimiden, Poly(oxyalkylen)carbamaten und poly(alkenyl)-N-substituierten Carbamaten, ausgewählt ist.

14. Verfahren zum Betrieb einer Brennkraftmaschine und zur Einschränkung des Anstiegs des Octanzahlbedarfs und/oder zur Verringerung des Octanzahlbedarfs und/oder zur Verringerung von Ablagerungen an den Einlaßventilen, bei dem man in der Maschine eine Kraftstoffzusammensetzung gemäß einem der Ansprüche 1 bis 13 verbrennt.

15. Für die Zugabe zu Benzin geeignetes Additivkonzentrat, das ein benzinverträgliches Verdünnungsmittel und eine Verbindung der Formel I gemäß einem der Ansprüche 1 bis 11 sowie gegebenenfalls noch mindestens ein weiteres Additiv gemäß Anspruch 13 enthält.

16. Verbindung der Formel I gemäß einem der Ansprüche 1 bis 11, wobei R₃ oder mindestens eine R₄-Gruppierung einen gegebenenfalls substituierten Kohlenwasserstoffrest mit mindestens vier Kohlenstoffatomen bedeutet.

17. Verfahren zur Herstellung einer Verbindung der Formel I gemäß Anspruch 16, bei dem man eine Verbindung der allgemeinen Formel XI 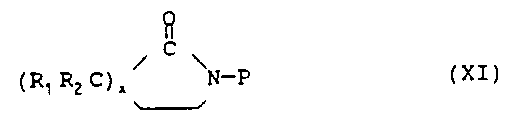 worin x, R₁ und R₂ die in Anspruch 1 angegebene Bedeutung haben und P Wasserstoff oder eine Gruppe der Formel -R₃-OH, worin R₃ die in Anspruch 1 angegebene Bedeutung hat, bedeutet, mit mindestens einem cyclischen Ether, von dem nach Ringöffnung eine Gruppierung -R₄-O-verbleibt, umsetzt, vorausgesetzt, daß man in dem Fall, daß P Wasserstoff bedeutet, die Verbindung der Formel XI mit mindestens einem mol eines cyclischen Ethers, von dem nach Ringöffnung eine Gruppierung -R₃-O- verbleibt, pro Mol der Verbindung der Formel XI zu einer Verbindung der Formel I, worin R₅ Wasserstoff bedeutet, umsetzt und gegebenenfalls anschließend durch Veretherung oder Veresterung in eine Verbindung der Formel I überführt, in der R₅ einen gegebenenfalls substituierten Kohlenwasserstoffrest mit 1 bis 100 Kohlenstoffatomen oder einen Acylrest mit 1 bis 20 Kohlenstoffatomen bedeutet.

## Revendications

1. Composition de carburant comprenant un mélange d'une quantité principale d'hydrocarbures bouillant dans la plage de l'essence et une quantité moindre d'un composé servant d'additif, répondant à la formule générale I : 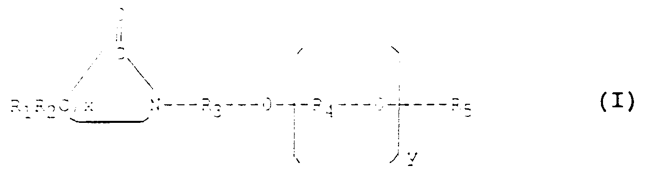 dans laquelle
x varie de 2 à 20; y varie de 1 à 50; R₁ et R₂ représentent, chacun indépendamment, un atome d'hydrogène ou un radical hydrocarbyle éventuellement substitué, comportant de 1 à 100 atomes de carbone; R₃ représente un radical hydrocarbyle éventuellement substitué comportant de 1 à 100 atomes de carbone; chaque symbole R₄ représente indépendamment un radical hydrocarbyle éventuellement substitué comportant de 2 à 100 atomes de carbone, de la formule : 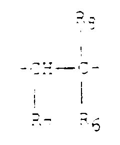 dans laquelle R₆, R₇ et R₈ représentent chacun indépendamment, un atome d'hydrogène, ou un radical hydrocarbyle éventuellement substitué, avec la condition qu'au moins l'un des symboles R₇ et R₈ représente un atome d'hydrogène, en sorte que R₆ et R₈ ensemble, ou R₆ et R₇ ensemble, contiennent de 0 à 98 atomes de carbone, ou que R₆ et R₈ ou R₆ et R₇, ensemble, représentent un pont hydrocarbyle contenant de 3 à 98 atomes de carbone; R₅ représente un atome d'hydrogène, ou un radical hydrocarbyle éventuellement substitué comportant de 1 à 100 atomes de carbone, ou un radical acyle comportant de 1 à 20 atomes de carbone et le poids de formule moyen du composé servant d'additif atteint au moins 600.

2. Composition de carburant suivant la revendication 1, caractérisée en ce que chacun des symboles R₆, R₇ et R₈ représente indépendamment un atome d'hydrogène ou un radical hydrocarbyle éventuellement substitué comportant de 1 à 18 atomes de carbone, avec la condition qu'au moins l'un des symboles R₇ et R₈ représente un atome d'hydrogène.

3. Composition de carburant suivant la revendication 1 ou 2, caractérisée en ce que le poids de formule moyen du composé servant d'additif varie de 800 à 4000.

4. Composition de carburant suivant l'une quelconque des revendications 1 à 3, caractérisée en ce que la valeur de x varie de 3 à 11.

5. Composition de carburant suivant l'une quelconque des revendications 1 à 4, caractérisée en ce que la valeur de y varie de 8 à 40.

6. Composition de carburant suivant l'une quelconque des revendications 1 à 5, caractérisée en ce que R₃ représente un radical hydrocarbyle comportant de 1 à 20 atomes de carbone.

7. Composition de carburant suivant l'une quelconque des revendications 1 à 6, caractérisée en ce que la valeur de x varie de 3 à 5, les symboles R₁ et R₂ représentent chacun indépendamment un atome d'hydrogène ou un radical hydrocarbyle comportant de 1 à 8 atomes de carbone; R₃ représente un radical hydrocarbyle comportant de 2 à 4 atomes de carbone; R₅ représente un atome d'hydrogène et chaque symbole R₆ représente indépendamment un atome d'hydrogène, un radical alkyle comportant de 1 à 18 atomes de carbone, ou un radical hydrocarbyle oxysubstitué comportant de 1 à 18 atomes de carbone et R₇ et R₈ représentent tous deux des atomes d'hydrogène.

8. Composition de carburant suivant la revendication 7, caractérisée en ce qu'un ou plusieurs symboles R₆ représentent des radicaux hydrocarbyle oxy-substitués, les symboles R₆ sont indépendamment choisis parmi les radicaux alkylène alcoxy-substitués comportant de 1 à 18 atomes de carbone, ou des radicaux alkylène aryloxysubstitués comportant de 1 à 18 atomes de carbone.

9. Composition de carburant suivant la revendication 7 ou 8, caractérisée en ce qu'un ou plusieurs symboles représentent des radicaux hydrocarbyle oxy-substitués, ces symboles R₆ étant indépendamment choisis parmi les suivants : éthylhexylèneoxyméthylène, isoproproxyméthylène, butoxyméthylène, nonylphénoxyméthylène et phénoxyméthylène.

10. Composition de carburant suivant l'une quelconque des revendications 1 à 7, caractérisée en ce que la valeur de x varie de 3 à 5; R₃ représente un radical hydrocarbyle comportant deux à quatre atomes de carbone; chaque symbole R₆ représenté indépendamment un atome d'hydrogène ou un radical hydrocarbyle comprenant des groupes alkyle de 1 à 2 atomes de carbone; R₇ et R₈ sont tous deux des atomes d'hydrogène et R₅ représente un atome d'hydrogène.

11. Composition de carburant suivant l'une quelconque des revendications 1 à 10, caractérisée en ce que R₁ et R₂ représentent chacun un atome d'hydrogène.

12. Composition de carburant suivant l'une quelconque des revendications 1 à 11, caractérisée en ce que le composé servant d'additif est présent en une proportion de 50 ppm en poids à 400 ppm en poids sur base du poids total de la composition de carburant.

13. Composition de carburant suivant l'une quelconque des revendications 1 à 12, caractérisée en ce qu'elle contient complémentairement une proportion mineure d'au moins un composé servant d'additif supplémentaire choisi dans le groupe formé par les polyalcénylamines, les amines de Mannich, les polyalcénylsuccinimides, les poly(oxyalkylène)carbamates et les carbamates poly(alcényle)-N-substitués.

14. Procédé pour faire fonctionner un moteur à combustion interne et contrôler l'augmentation du besoin en octane et/ou réduire le besoin en octane et/ou réduire les dépôts sur des soupapes d'admission, caractérisé en ce que l'on brûle dans le moteur précité une composition de carburant suivant l'une quelconque des revendications 1 à 13.

15. Concentré d'additif convenant à l'addition à de l'essence, caractérisé en ce qu'il comprend un diluant compatible avec l'essence et un composé de la formule I, tel que défini dans l'une quelconque des revendications 1 à 11, et éventuellement aussi au moins un composé servant d'additif supplémentaire, tel que défini dans la revendication 13.

16. Composé de la formule I suivant l'une quelconque des revendications 1 à 11, dans lequel R₃ ou au moins un groupement R₄ représente un radical hydrocarbyle éventuellement substitué possédant au moins quatre atomes de carbone.

17. Procédé de préparation d'un composé de la formule I, tel que défini dans la revendication 16, caractérisé en ce que l'on fait réagir un composé de la formule générale XI : 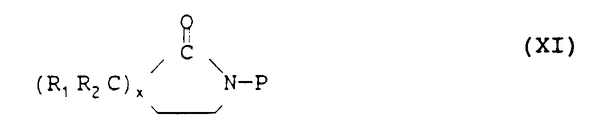 dans laquelle x, R₁ et R₂ possèdent les significations qui leur ont été attribuées dans la revendication 1 et P représente un atome d'hydrogène ou un groupe de la formule -R₃-OH, où R₃ possède les significations qui lui ont été attribuées dans la revendication 1, avec au moins un composé du type éther cyclique, qui, par ouverture du cycle, donne un groupement -R₄-O-, avec la condition que lorsque P représente un atome d'hydrogène, on fait réagir le composé de la formule XI avec au moins 1 mole du composé du type éther cyclique, qui donne un groupement -R₃-O- par ouverture du cycle, par mole du composé de la formule XI, pour former un composé de la formule I, dans laquelle R₅ représente un atome d'hydrogène, puis on procède à une éthérification ou une estérification éventuelle pour former un composé de la formule I, dans laquelle R₅ représente un radical hydrocarbyle éventuellement substitué comportant 1 à 100 atomes de carbone, ou un radical acyle comportant 1 à 20 atomes de carbone.
